# EUROPEAN PATENT SPECIFICATION

(11) **EP 1 687 856 B1**
(45) Date of publication and mention of the grant of the patent: **28.02.2007**
(21) Application number: 04816330.7
(22) Date of filing: 17.11.2004
(51) Int. Cl.: H01L 51/00, C09K 11/06, C07D 241/12

(54) **ELECTROLUMINESCENT DEVICES COMPRISING 2-(P-TRIPHENYL)-3-PHENYL-PYRAZINE DERIVATIVES**
ELEKTROLUMINESZENTE BAUELEMENTE MIT 2-(P-TRIPHENYL)-3-PHENYL-PYRAZINDERIVATEN
APPAREILS ELECTROLUMINESCENTS CONTENANT DERIVES DE LA 2-(P-TRIPHENYL)-3-PHENYL-PYRAZINE

(30) Priority: 26.11.2003 EP 03104389
(43) Date of publication of application: 09.08.2006
(73) Proprietor: Ciba Specialty Chemicals Holding Inc., 4057 Basel (CH)
(72) Inventor: SCHÄFER, Thomas, CH-4053 Basel (CH); PEUCHMAUR, Marine, F-38140 Apprieu (FR); ROGERS, Jonathan, White Plains, NY 10606 (US); CRAIG, Michael, Robert, New York, NY 10016 (US)
(86) International application number: PCT/EP2004/052984
(87) International publication number: WO 2005/053048

(56) References cited:
- WO-A-02/088274
- US-A- 5 077 142

## Description

The present invention relates to organo-electroluminescent (EL) devices, in particular EL devices that comprise durable, blue-emitting organo-electroluminescent layers. The organo-electroluminescent layers comprise certain pyrazine compounds.

The present invention is aimed at an electroluminescent device comprising an organic light-emitting layer that contains at least one blue-emitting pyrazine compound.

JP09188875A relates to a luminescent element comprising between an anode and a cathode a hole transport layer/electron transfer layer, a luminescent layer/electron transport layer or a monolayer structure made of a mixture of a luminescent material and an electron transport material and/or hole transporting material. An aromatic compound represented by the formula 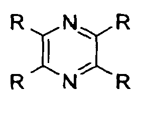 (wherein at least one R is an aromatic substituent and the number of nitrogen atoms of the substituents R is at least 1) having at least one six-membered ring structure and at least three nitrogen atoms in the molecule is vapor deposited on the electron transfer layer sandwiched between the anode and the cathode.

In US-A-5077142 an organic EL device is described comprising 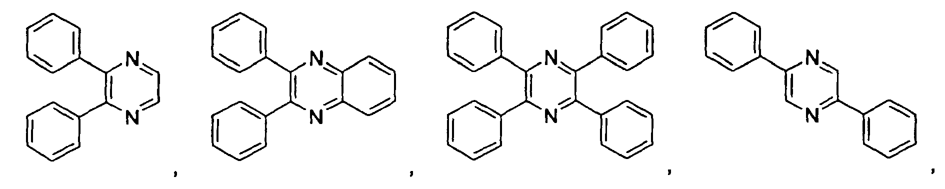 or 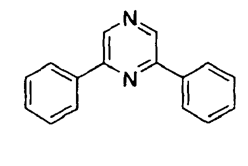 (see also JP06088072).

JP08022040 discloses an organic non-linear optical material of formula 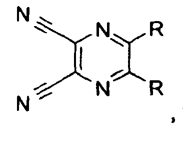 wherein R is H, optionally substituted alkyl, alkoxy, optionally substituted aryl or aryloxy, optionally substituted alkylthio, alkylcarbonyloxy, alkylthiocarbonyloxy, OH, or halogen.

JP2003086381A, JP09188875A, JP2003040873A, JP1997188875A disclose EL devices,
wherein quinoxaline compounds, such as 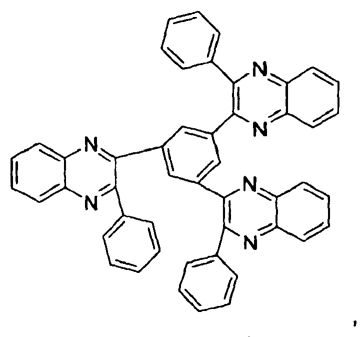 are used in the electron transport layer and/or electron injection layer.

JP2003109763A relates to EL devices, comprising the following pyrazine compound:

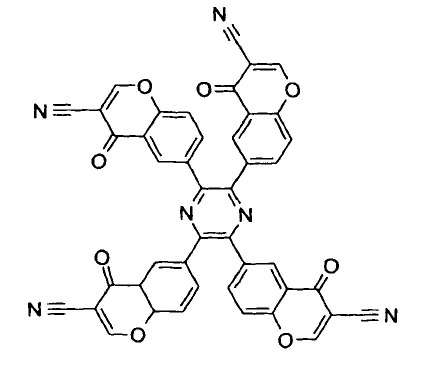

EP-A-763965 relates to blue emitting materials of formula 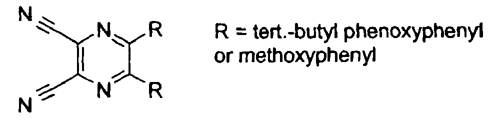 and EL devices containing these materials.

EP-A-1148109 relates to EL devices, wherein among others quinoxaline compounds are used as host compounds.

WO02/088274 relates to EL devices, comprising double-spiro organic compounds, such as,, for example, chemical compound 209:

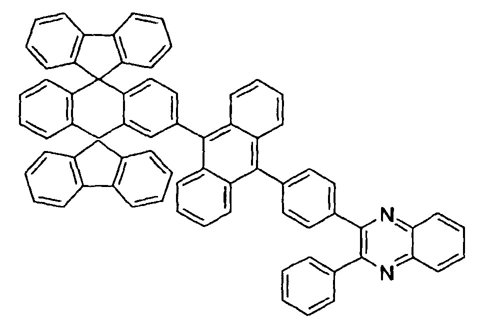

It is the object of the present invention to provide a light emitting element with excellent light emitting characteristics and durability.

Accordingly the present invention relates to an electroluminescent device, comprising a pyrazine compound of formula I.

In a preferred embodiment the electroluminescent device comprises in this order
(a) an anode
(b) a hole injecting layer and/or a hole transporting layer
(c) a light-emitting layer
(d) optionally an electron transporting layer and
(e) a cathode, wherein the pyrazine compound of formula I is preferably contained in the light-emitting layer.

In addition the present invention is also directed to the use of the pyrazine compounds of formula I for electrophotographic photoreceptors, photoelectric converters, solar cells, image sensors, dye lasers and electroluminescent devices.

The pyrazine compounds of formula I are novel and form a further object of the present invention.

Accordingly, the present invention relates also to pyrazine compounds of formula 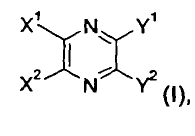 wherein
X¹ is a group of formula 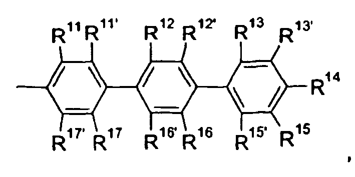 or a C₁₆-C₃₀aryl group, which can optionally be substituted by E;
X² is an aryl group, or a heteroaryl group, which can optionally be substituted; especially a group of formula 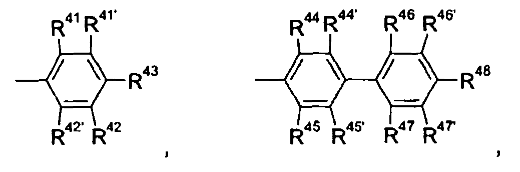 or 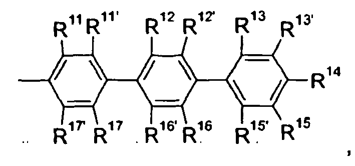 or a C₁₆-C₃₀aryl group, which can optionally be substituted by E;
Y¹ and Y² are independently of each other a hydrogen atom, C₁-C₁₈alkyl, which is optionally interrupted by O,
   an aryl group or a heteroaryl group, which can optionally be substituted; especially a C₁₆-C₃₀aryl group, which can optionally be substituted by E; or a group of formula 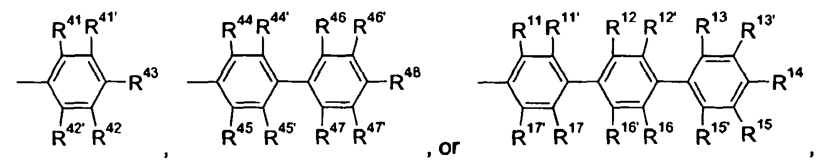 or
Y¹ and Y² together form a C₅-C₈cycloalkyl group, wherein
R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ and R^{47'} are independently of each other H, E, C₆-C₁₈aryl; C₆-C₁₈aryl which is substituted by E; C₁-C₁₈alkyl; C₁-C₁₈alkyl which is substituted by E' and/or interrupted by D; C₇-C₁₈aralkyl; or C₇-C₁₈aralkyl which is substituted by E; or
R^{11'} and R¹², R^{12'} and R¹³, R^{15'} and R¹⁶, R^{16'} and R¹⁷, R^{44'} and R⁴⁶ and/or R⁴⁵ and R⁴⁷ are each a divalent group L¹ selected from an oxygen atom, an sulfur atom, >CR¹⁸R¹⁹ >SiR¹⁸R¹⁹, or 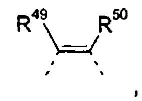 wherein
R¹⁸ and R¹⁹ are independently of each other C₁-C₁₈alkyl; C₁-C₁₈alkoxy, C₆-C₁₈aryl, C₆-C₁₈aryl, which is substituted by E; C₇-C₁₈aralkyl, or C₇-C₁₈aralkyl, which is substituted by E; or
R¹¹ and R^{11'}, R¹² and R^{12'}, R¹³ and R^{13'}, R^{13'} and R¹⁴, R¹⁴ and R¹⁵, R¹⁵ and R^{15'}, R¹⁶ and R^{16'}, R^{17'} and R¹⁷, R⁴¹ and R^{41'}, R⁴² and R^{42'}, R^{42'} and R⁴³, R^{41'} and R⁴³, R⁴⁴ and R^{44'}, R⁴⁵ and R^{45'}, R⁴⁶ and R^{46'}, R⁴⁷ and R^{47'}, R^{46'} and R⁴⁸ and/or R^{47'} and R⁴⁸ are each a divalent group 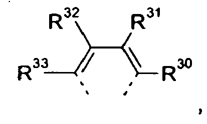 wherein
R³⁰, R³¹, R³², R³³, R⁴⁹ and R⁵⁰ are independently of each other H, C₁-C₁₈alkyl; C₁-C₁₈alkyl, which is substituted by E' and/or interrupted by D; E; C₆-C₁₈aryl; C₆-C₁₈aryl, which is substituted by E;
R¹⁴ is H, C₂-C₃₀heteroaryl, -NR⁷⁰R⁷¹, C₆-C₃₀aryl, or C₆-C₃₀aryl which is substituted by E, C₁-C₁₈alkyl; or C₁-C₁₈alkyl which is substituted by E' and/or interrupted by D; especially 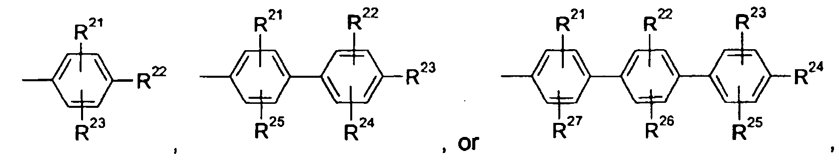 wherein R²¹, R²², R²³, R²⁴, R²⁵, R²⁶ and R²⁷ are independently of each other H, E, C₁-C₁₈alkyl; C₁-C₁₈alkyl which is substituted by E' and/or interrupted by D; E; C₇-C₁₈aralkyl; C₇-C₁₈aralkyl which is substituted by E;
R⁴³ and R⁴⁸ are independently of each other H, E; especially C₁-C₂₄alkyl, C₁-C₂₄alkoxy, or -NR⁷⁰R⁷¹, wherein R⁷⁰ and R⁷¹ are independently of each other H, C₆-C₁₈aryl, C₆-C₁₈aryl which is substituted by C₁-C₂₄alkyl, or C₁-C₂₄alkoxy, C₁-C₂₄alkyl, or C₁-C₂₄alkyl which is interrupted by -0-, or
R⁷⁰ and R⁷¹ together form a five or six membered ring, in particular 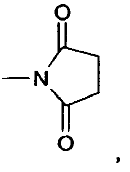 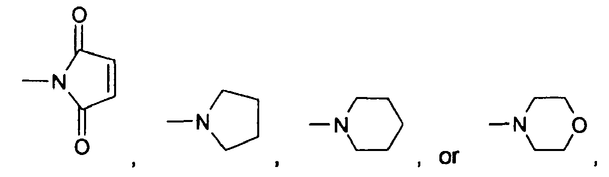 C₁-C₁₈alkyl; C₁-C₁₈alkyl, which is substituted by E and/or interrupted by D; C₂-C₃₀heteroaryl; C₇-C₁₈aralkyl; C₇-C₁₈aralkyl which is substituted by E;
D is -CO-; -COO-; -OCOO-; -S-; -SO-; -SO₂-; -O-; -NR⁵-; -SiR⁶¹R⁶²-; -POR⁵-; -CR⁶³=CR⁶⁴-; or -C≡C-;
E is C₁-C₁₈alkyl, -OR⁵; -SR⁵; -NR⁵R⁶; -COR⁸; -COOR⁷; -CONR⁵R⁶; -CN; or halogen;
E' is E, except C₁-C₁₈alkyl, wherein
R⁵ and R⁶ are independently of each other C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -O-; or
R⁵ and R⁶ together form a five or six membered ring, in particular 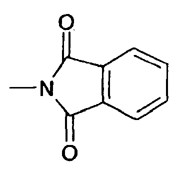 or 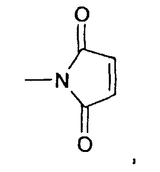
R⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-;
R⁸ is C₇-C₁₂alkylaryl; C₁-C₁₈alkyl; or C₁-C₁₈alkyl which is interrupted by -0-;
R⁶¹ and R⁶² are independently of each other C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -0-, and
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -0-.
R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, and R^{47'} as well as R¹⁴, R⁴³, and R⁴⁸ are preferably independently of each other H, E; or C₁-C₈alkyl, especially H, C₁-C₄alkyl, C₁-C₄alkoxy, or phenyl; wherein E is -OR⁵; -SR⁵; -NR⁵R⁶; -COR⁸; -COOR⁷; -CONR⁵R⁶; -CN; -OCOOR⁷; or halogen, especially F; wherein R⁵ and R⁶ are independently of each other C₆-C₁₂ary, or C₁-C₈alkyl;
R⁷ is C₇-C₁₂ alkylaryl, or C₁-C₈alkyl; and
R⁸ is C₆-C₁₂aryl; or C₁-C₈alkyl, or
R¹¹ and R^{11'}, R¹² and R^{12'}, R¹³ and R^{13'}, R^{13'} and R¹⁴, R⁴¹ and R^{41'}, R^{41'} and R⁴³, R⁴⁴ and R^{44'},
R⁴⁶ and R^{46'}, R^{46'} and R⁴⁸ and/or R⁴⁷ and R⁴⁸ are each a divalent group

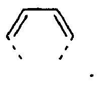

According to the present invention at least X¹, preferably X¹ and X² are a group of formula 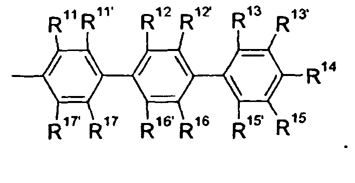 X¹ and X² can be different, but are preferably the same.

Preferably X¹ and X² are independently of each other a group of formula 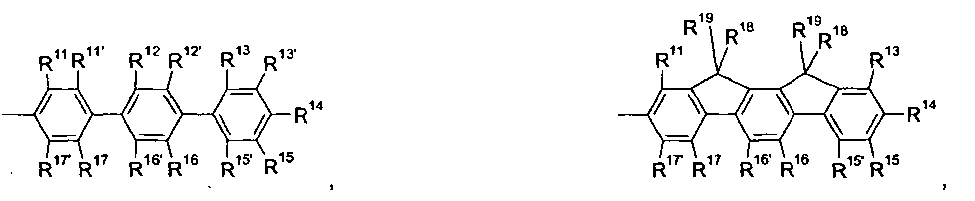 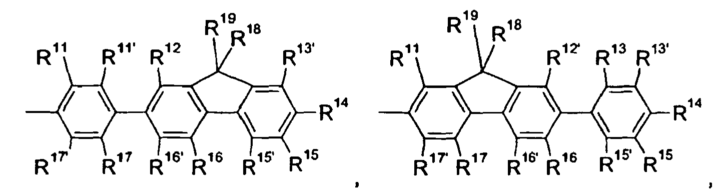 or -X¹¹-X¹²-X¹³, wherein
R¹¹, R^{11'}, R¹², R^{12'}, R¹³, R^{13'}, R¹⁵, R^{15'}, R¹⁶, R^{16'}, R¹⁷ and R^{17'} are independently of each other H, C₆-C₁₈aryl; C₆-C₁₈aryl which is substituted by E; E, C₁-C₁₈alkyl; C₁-C₁₈alkyl which is substituted by E' and/or interrupted by D; C₇-C₁₈aralkyl; C₇-C₁₈aralkyl which is substituted by E; and
R¹⁴, R¹⁸ and R¹⁹ are as defined above,
X¹¹ and X¹² are independently of each other a group of formula 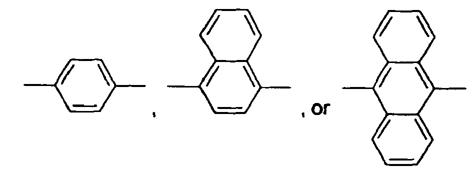 and X¹³ is a group of formula 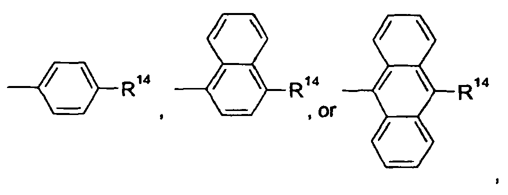 wherein R¹⁴ is 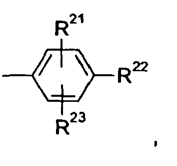 or 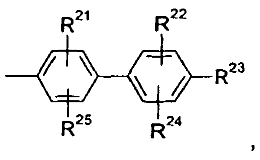 wherein R²¹, R²², R²³, R²⁴ and R²⁵ are as defined above and Y¹ and Y² are a hydrogen atom, C₁-C₁₈alkyl, which is optionally interrupted by O, or Y¹ and Y² together form a C₅-C₈cycloalkyl group, and are especially a hydrogen atom.

In a preferred embodiment X¹ and X² are a group of formula 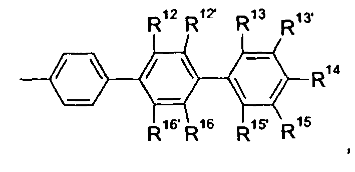 wherein
R¹³, R^{13'}, R¹⁵ and R^{15'} are H and R¹⁴ is H, or 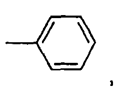 and R¹², R^{12'}, R¹⁶ and R^{16'} are H; or R¹³ and R¹⁵ are H, R^{13'} and R^{15'} are independently of each other H, C₁-C₈alkyl, or C₁-C₈alkoxy, and R¹⁴ is H, C₁-C₈alkyl, or C₁-C₈alkoxy, and R¹², R^{12'}, R¹⁶ and R^{16'} are H, wherein at least one of R¹³, R¹⁵, R^{13'}, R^{15'} and R¹⁴ is C₁-C₈alkyl, or C₁-C₈alkoxy; or
R¹² and R^{12'}, R¹³ and R^{13'}, R^{13'} and R¹⁴, R¹⁴ and R¹⁵, R¹⁵ and R^{15'}, and/or R¹⁶ and R^{16'}, are a divalent group 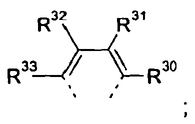 or
R^{12'}, R¹⁶, R^{16'} are H and R¹³ and R^{13'}, and/or R^{13'} and R¹⁴ are a divalent group 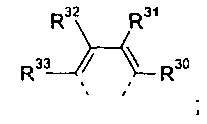 or
R¹³, R^{13'}, R¹⁴, R¹⁵, R^{15'} are H and R¹² and R¹², and/or R¹⁶ and R^{16'} are a divalent group 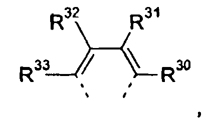 wherein R³⁰, R³¹, R³² and R³³ are H, C₁-C₈alkyl, or C₁-C₈alkoxy, and
Y¹ and Y² are a hydrogen atom.

In a preferred embodiment X¹ and X² are independently of each other a group of formula 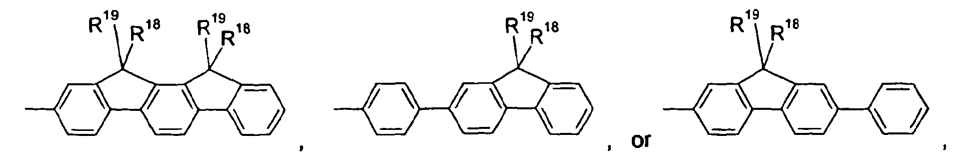 wherein R¹⁸ and R¹⁹ are independently of each other C₁-C₈alkyl.

In a preferred embodiment X¹ is a group of formula 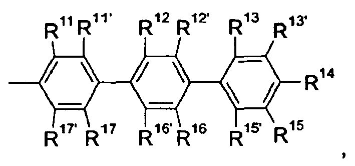 especially 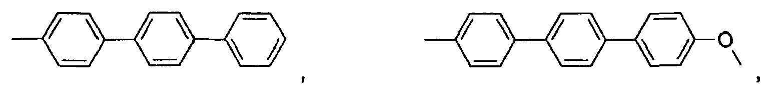 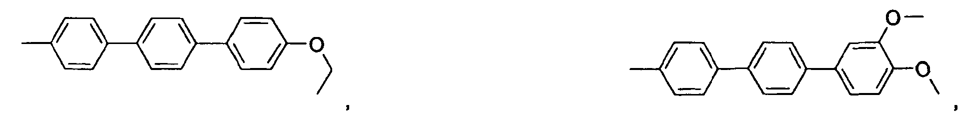 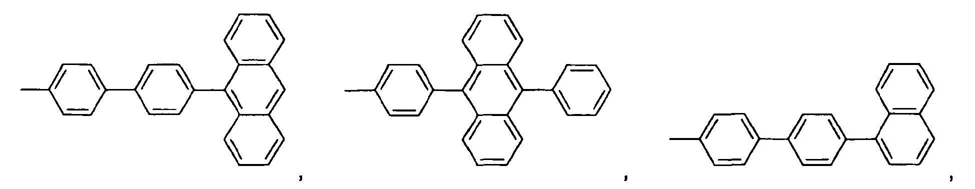 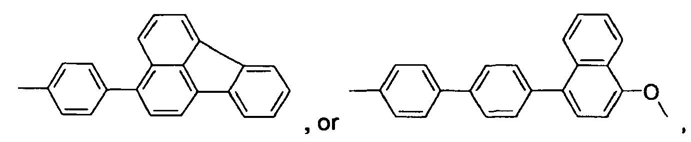 and X² is a group of formula 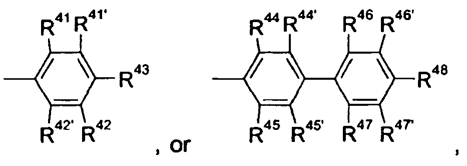 especially 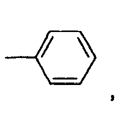 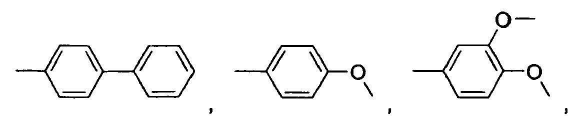 or 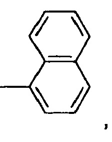 in particular a group of formula 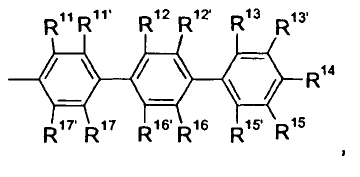 such as 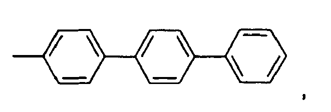 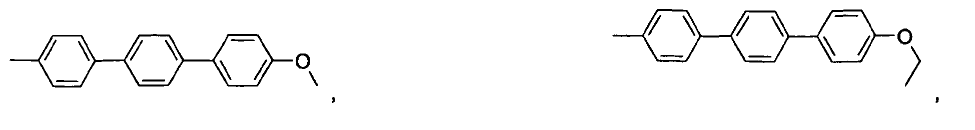 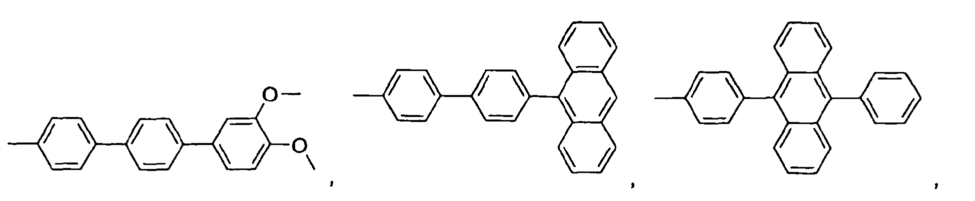 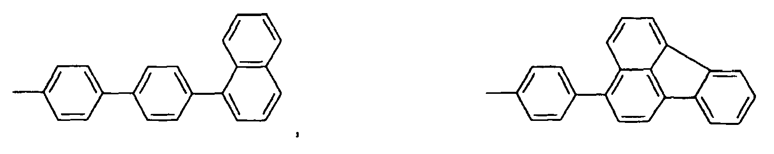 or 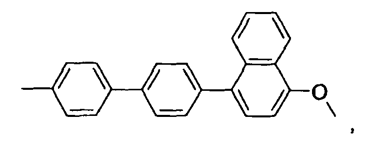
wherein R¹¹, R^{11'}, R¹², R^{12'}, R¹³, R^{13'}, R¹⁴, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, R^{47'}, R⁴³ and R⁴⁸ are as defined above and are especially H, C₁-C₈alkyl, C₁-C₈alkoxy, or phenyl, or
R¹³ and R^{13'}, R^{13'} and R¹⁴, R¹⁴ and R¹⁵, or R¹⁵ and R^{15'} can be a divalent group 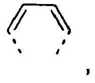 and Y¹ and Y² are a hydrogen atom.
R¹¹, R^{11'}, R¹², R^{12'}, R¹³, R^{13'}, R¹⁵, R^{15'}, R¹⁶, R^{16'}, R¹⁷ and R^{17'}, R⁴¹, R^{41'}, R⁴², R^{42'}, R⁴⁴, R^{44'}, R⁴⁵, R^{45'}, R⁴⁶, R^{46'}, R⁴⁷, and R^{47'} as well as R¹⁴, R⁴³, and R⁴⁸ are preferably independently of each other H, E; or C₁-C₈alkyl; wherein E is -OR⁵; -SR⁵; -NR⁵R⁶; -COR⁸; -COOR⁷; -CONR⁵R⁶; -CN; -OCOOR⁷; or halogen; wherein R⁵ and R⁶ are independently of each other C₆-C₁₂aryl, or C₁-C₈alkyl; R⁷ is C₇-C₁₂alkylaryl, or C₁-C₈alkyl; and R⁸ is C₆-C₁₂aryl; or C₁-C₈alkyl.

If X¹ and/or X² as well as Y¹ and/or Y² are a C₁₆-C₃₀aryl group, they are especially a fluoranthenyl, triphenlenyl, chrysenyl, naphthacen, picenyl, perylenyl, such as 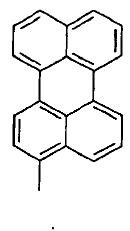 or 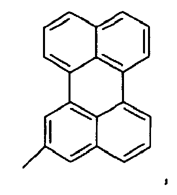 pentaphenyl, hexacenyl, or pyrenyl group, which can be substituted by E; very especially a fluoranthenyl group, which can be substituted by E.

Accordingly, in a further preferred embodiment the present invention is directed to compounds of formula I, wherein Y¹ and Y² are hydrogen and X¹ and X² are independently of each other a group Ar¹-Ar², wherein
Ar¹ is a group of formula 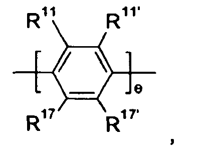
Ar² is a group of formula 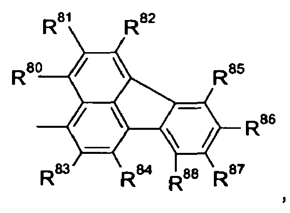 wherein
R⁸⁰, R⁸¹, R⁸², R⁸³, R⁸⁴, R⁸⁵, R⁸⁶, R⁸⁷ and R⁸⁸ are independently of each other H, E', C₆-C₁₈aryl; C₆-C₁₈aryl, which is substituted by E; C₁-C₁₈alkyl; C₁-C₁₈alkyl which is substituted by E' and/or interrupted by D; C₇-C₁₈aralkyl; or C₇-C₁₈aralkyl which is substituted by E;
e is an integer 1, or 2, and R¹¹, R^{11'}, R¹⁷ and R^{17'} are defined as above.

In said embodiment compounds of formula I are especially preferred, wherein
X¹ and X² are a group Ar¹-Ar², wherein
Ar¹ is a group of formula 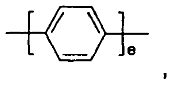
Ar² is a group of formula 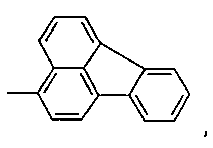 and
e is an integer 1, or 2.

In a further preferred embodiment the present invention is directed to compounds of formula I, wherein Y¹ and Y² are independently of each other a group of the formula - W¹ -(W²)_{b}-W³, wherein b is 0, or, 1, especially hydrogen, and X¹ and X² are independently of each other a group -W¹ -(W²)_{b}-W³, wherein
W¹ and W² are independently of each other a group of formula 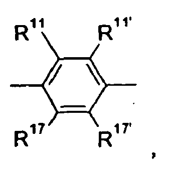 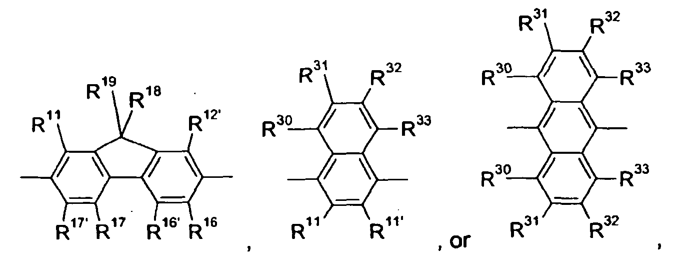
W³ is a group of formula 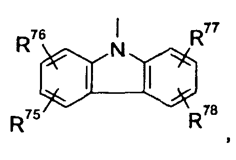 or-NR⁷⁰R⁷¹, wherein R⁷⁰ and R⁷¹ are independently of each other a group of formula 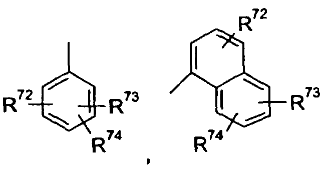 or 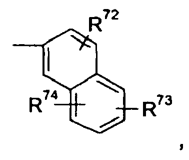 wherein R⁷², R⁷³ and R⁷⁴ are independently of each other hydrogen, C₁-C₈alkyl, C₁-C₈alkoxy, C₁-C₈alkylthio, a cyano group, a carbamoyl group, an amino group, a silyl group or a siloxanyl group,
R⁷⁵, R⁷⁶, R⁷⁷ and R⁷⁸ are independently of each other H, E, C₆-C₁₈aryl; C₆-C₁₈aryl which is substituted by C₁-C₁₈alkoxy, C₁-C₁₈alkyl, C₁-C₁₈alkyl which is interrupted by -O-; C₇-C₁₈aralkyl; or C₇-C₁₈aralkyl which is substituted by C₁-C₁₈alkoxy; wherein E, R¹¹, R^{11'}, R^{12'}, R¹⁶, R^{16'}, R¹⁷, R^{17'}, R¹⁸, R¹⁹, R³⁰, R³¹, R³² and R³³ are as defined above.

In said embodiment compounds of formula I are especially preferred, wherein
Y¹ and Y² are hydrogen,
X¹ and X² are a group of the formula -W¹ -(W²)b-W³, wherein b is 0, or 1,
W¹ is a group of formula 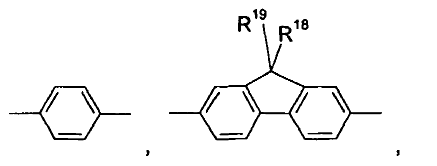
W² is a group of formula 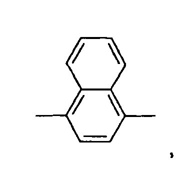 or 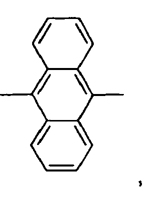
W³ is a group of formula 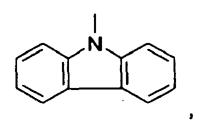 or -NR⁷⁰R⁷¹, wherein R⁷⁰ and R⁷¹ are independently of each other a group of formula 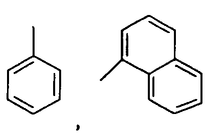 or 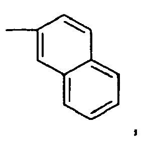 and
R¹⁸ and R¹⁹ are independently of each other C₁-C₁₈alkyl.

In a particularly preferred embodiment of the present invention the pyrazine is a compound of formula I, wherein X¹ is a group of formula 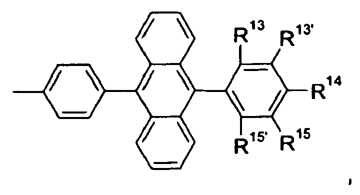 wherein R¹³, R^{13'}, R¹⁴, R¹⁵ and R^{15'} are independently of each other H, C₁-C₈alkyl, especially methyl, ethyl, n-butyl, t-butyl, C₁-C₈alkoxy, especially methoxy, ethoxy, butoxy, phenyl, phenoxy, or R¹³ and R^{13'} or R^{13'} and R¹⁴ are a divalent group

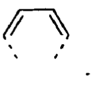

In a preferred embodiment of the present invention the pyrazine is a compound of formula I, wherein Y¹ and Y² are hydrogen. X¹ and X² can be different, but are preferably the same.

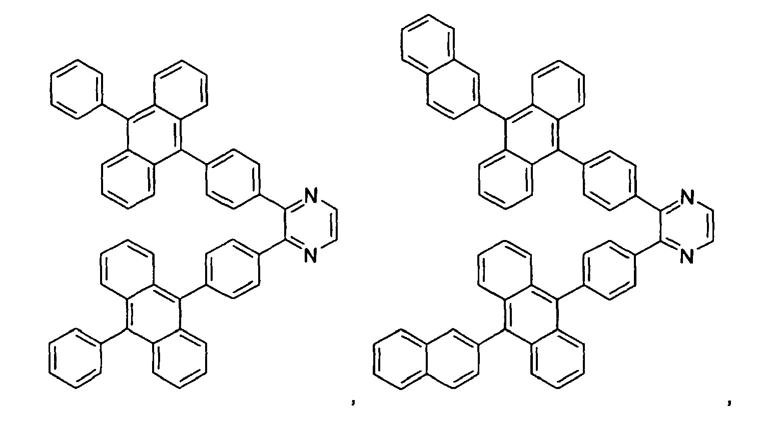

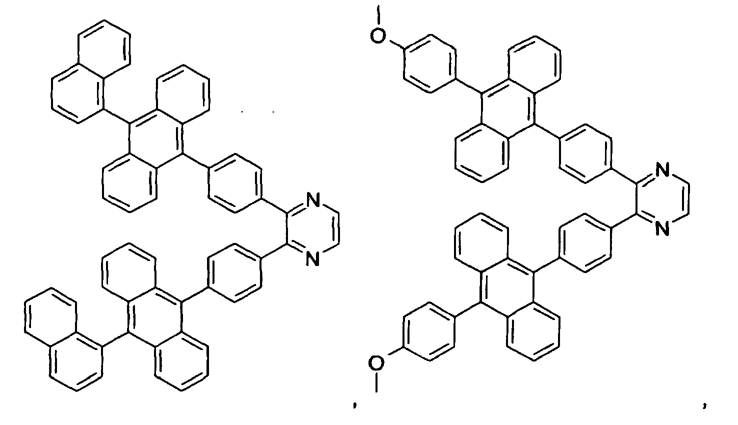

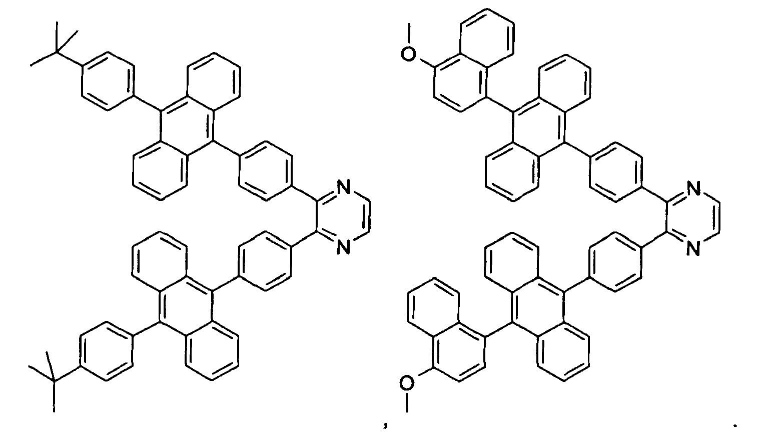

In a preferred embodiment of the present invention the pyrazine is a compound of formula I, wherein
X¹ is a group of formula 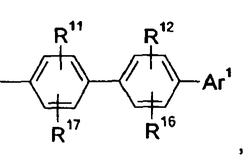 wherein Ar¹ is a group of formula 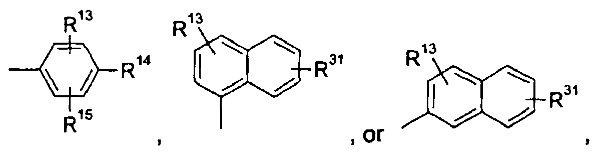
X² is a group of formula 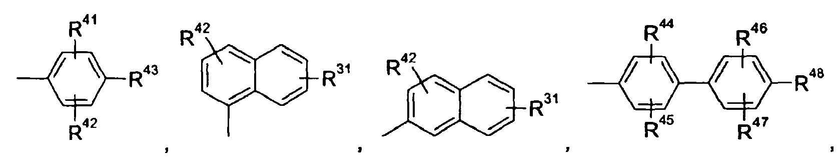 or 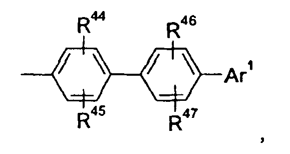
Y¹ and Y² are independently of each other H, C₁-C₈alkyl, or Ar², wherein
R¹¹, R¹², R¹³, R¹⁵, R¹⁶, R¹⁷, R³¹, R⁴¹, R⁴², R⁴⁴, R⁴⁵, R⁴⁶ and R⁴⁷ are independently of each other H, -OR⁵, -NR⁶R^{6'}, C₁-C₈alkyl, or phenyl,
R¹⁴ is H, -OR⁵, -NR⁶R^{6'}, or C₁-C₈alkyl,
R⁴³ and R⁴⁸ are independently of each other H, -OR⁵, -NR⁶R^{6'}, C₁-C₈alkyl, or phenyl,
R⁵ is C₁-C₈alkyl, or phenyl, and
R⁶ and R⁶ are independently of each other C₁-C₈alkyl.

Specific examples of preferred pyrazine compounds are given below: 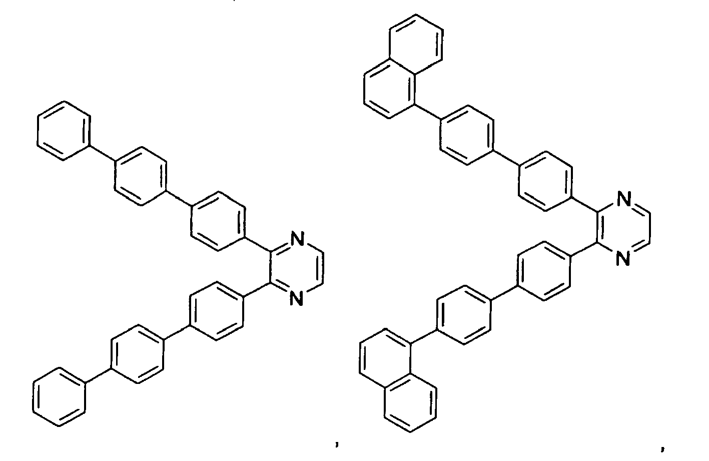 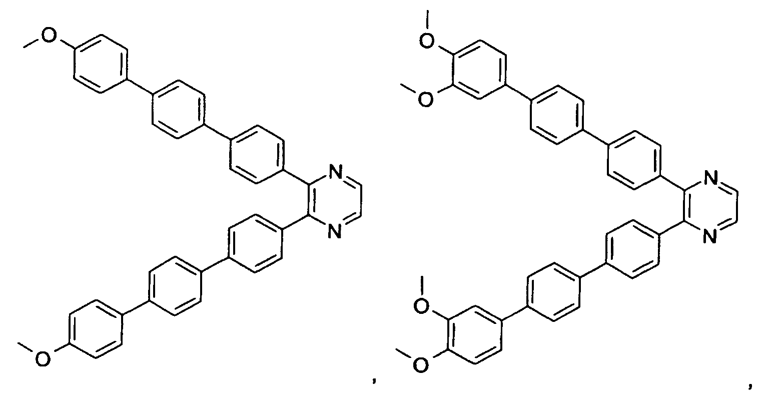 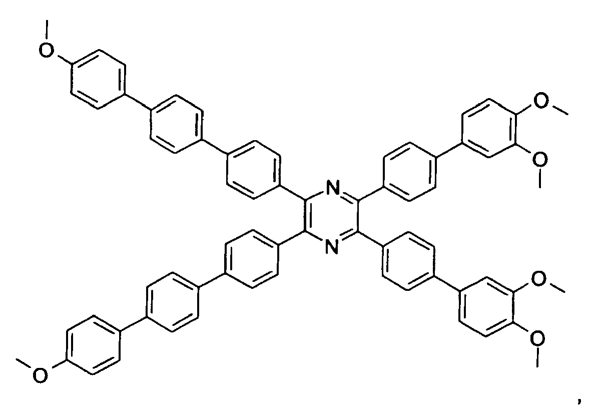 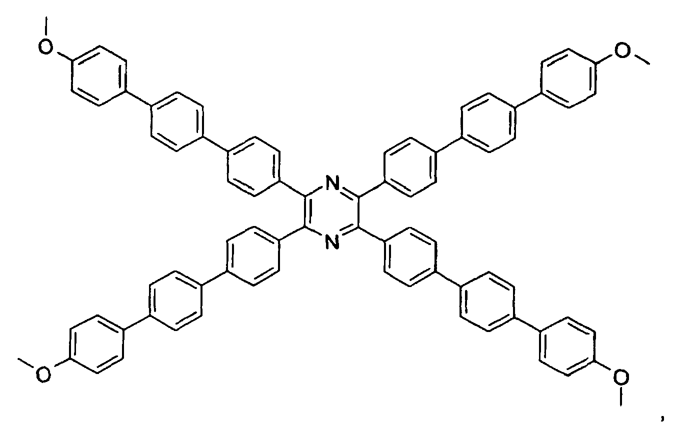 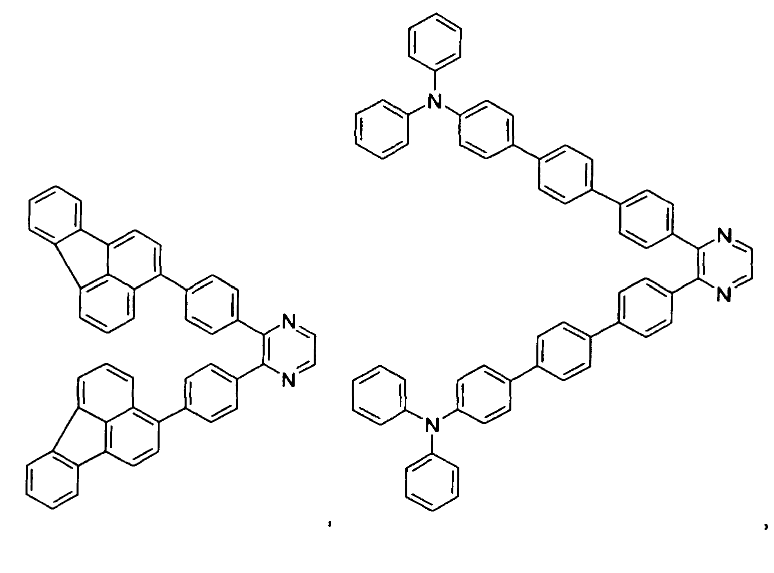 and

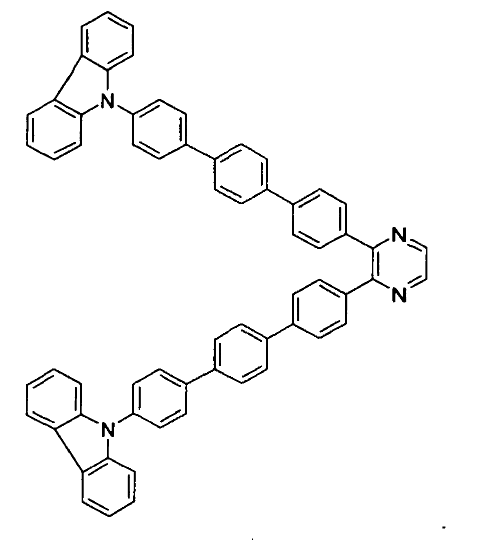

The present pyrazine compounds can be prepared according to or analogous to known procedures. The pyrazine compounds of the present invention of the formula: 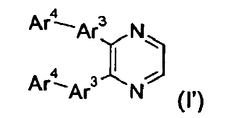 can, for example, be prepared according to a process (N. Miyaua and A. Suzuki in Chemical Reviews, Vol. 95, pp. 457-2483 (1995), which comprises reacting a derivative of formula 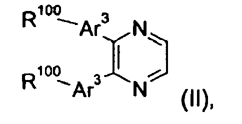 wherein R¹⁰⁰ stands for halogen such as chloro or bromo, preferably bromo, or E¹ having the meaning of 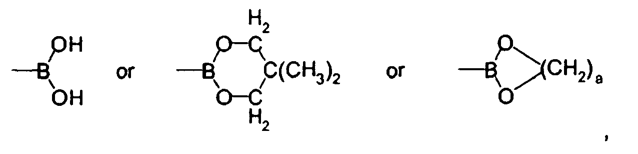 wherein a is 2 or 3,
with boronic acid derivative
E¹-Ar⁴,
or - in case R¹⁰⁰ is not halogen -
Hal-Ar⁴,
wherein Hal stands for halogen, preferably for bromo,
wherein Ar³ is a group of formula 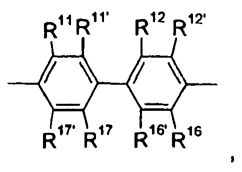 and Ar⁴ is a group of formula 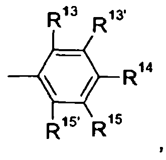 in the presence of a palladium catalyst, especially an allylpalladium catalyst of the µ-halo(triisopropylphosphine)(η³-allyl)palladium(II) type (see for example WO99/47474). The reaction is typically conducted at about 70 °C to 120 °C in an aromatic hydrocarbon solvent such as toluene. Other solvents such as dimethylformamide and tetrahydrofuran can also be used alone, or in mixtures with an aromatic hydrocarbon. An aqueous base, preferably sodium carbonate or bicarbonate, is used as the HBr scavenger. Depending on the reactivities of the reactants, a polymerization reaction may take 2 to 100 hours. Organic bases, such as, for example, tetraalkylammonium hydroxide, and phase transfer catalysts, such as, for exampleTBAB, can promote the activity of the boron (see, for example, Leadbeater & Marco; Angew. Chem. Int. Ed., 2003, 42, 1407 and references cited therein). Other variations of reaction conditions are given by T. I. Wallow and B. M. Novak in Journal of Organic Chemistry, Vol. 59, pp. 5034-5037 (1994); and M. Remmers, M. Schulze, and G. Wegner in Macromolecular Rapid Communications, Vol. 17, pp. 239-252 (1996).

The compound of formual II can, for example, be obtained by reacting a compound of formula V and ethylene diamine and oxidizing the obtained compound of formual IV with DDQ. 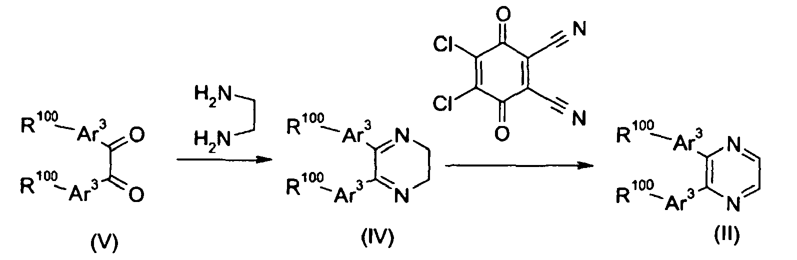 (Akihiro Ohta et al., J. Heterocyclic Chem. 21 (1984) 103-106).

Accordingly, tetrasubstituted pyrazine compounds of the present invention of the formula: 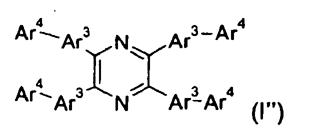 can, for example, be prepared by reacting a compound of formula 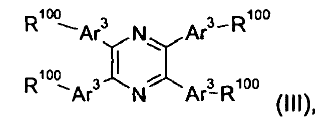 with a boronic acid derivative
E¹-Ar⁴, or - in case R¹⁰⁰ is not halogen - Hal-Ar⁴ in the presence of an allylpalladium catalyst of the µ-halo(triisopropylphosphine)(η³-allyl)palladium(II) type.

The intermediates of formula II and III, such as 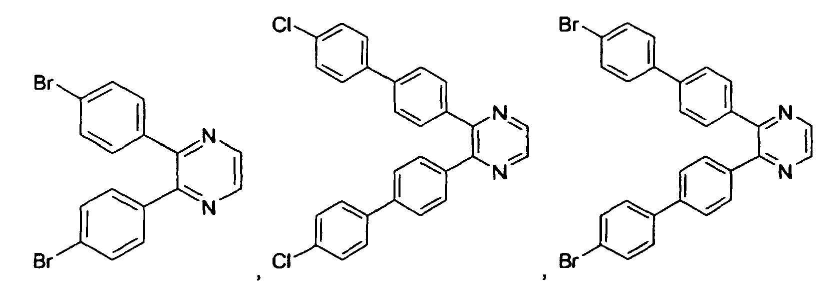 are novel and form a further object of the present application.

C₁-C₁₈alkyl is a branched or unbranched radical such as for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl, dodecyl, 1,1,3,3,5,5-hexamethylhexyl, tridecyl, tetradecyl, pentadecyl, hexadecyl, heptadecyl, or octadecyl.
C₁-C₁₈Alkoxy radicals are straight-chain or branched alkoxy radicals, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy.

C₂-C₁₈Alkenyl radicals are straight-chain or branched alkenyl radicals, such as e.g. vinyl, allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

C₂₋₂₄Alkynyl is straight-chain or branched and preferably C₂₋₈alkynyl, which may be unsubstituted or substituted, such as, for example, ethynyl, 1-propyn-3-yl, 1-butyn-4-yl, 1-pentyn-5-yl, 2-methyl-3-butyn-2-yl, 1,4-pentadiyn-3-yl, 1,3-pentadiyn-5-yl, 1-hexyn-6-yl, cis-3-methyl-2-penten-4-yn-1-yl, trans-3-methyl-2-penten-4-yn-1-yl, 1,3-hexadiyn-5-yl, 1-octyn-8-yl, 1-nonyn-9-yl, 1-decyn-10-yl, or 1-tetracosyn-24-yl.

C₄-C₁₈cycloalkyl is preferably C₅-C₁₂cycloalkyl, such as, for example, cyclopentyl, cyclohexyl, cycloheptyl, cyclooctyl, cyclononyl, cyclodecyl, cyclododecyl. Cyclohexyl and cyclododecyl are most preferred.

The term "aryl group" is typically C₆-C₃₀aryl, such as phenyl, indenyl, azulenyl, naphthyl, biphenyl, terphenylyl or quadphenylyl, as-indacenyl, s-indacenyl, acenaphthylenyl, phenanthryl, fluoranthenyl, triphenlenyl, chrysenyl, naphthacen, picenyl, perylenyl, pentaphenyl, hexacenyl, pyrenyl, or anthracenyl, preferably phenyl, 1-naphthyl, 2-naphthyl, 9-phenanthryl, 2- or 9-fluorenyl, 3- or 4-biphenyl, which may be unsubstituted or substituted. Examples of C₆-C₁₈aryl are phenyl, 1-naphthyl, 2-naphthyl, 3- or 4-biphenyl, 9-phenanthryl, 2- or 9-fluorenyl, which may be unsubstituted or substituted.

C₇-C₂₄aralkyl radicals are preferably C₇-C₁₈aralkyl radicals, which may be substituted, such as, for example, benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted.

C₇-C₁₂alkylaryl is, for example, a phenyl group substituted with one, two or three C₁-C₈alkyl groups, such as, for example, 2-, 3-, or 4-methylphenyl, 2-, 3-, or 4 -ethylphenyl, 3-, or 4-isopropylphenyl, 3,4-dimethylphenyl, 3,5-dimethylphenyl, or 3,4,5-trimethylphenyl.

The term "heteroaryl group", especially C₂₋C₃₀heteroaryl, is a ring, wherein nitrogen, oxygen or sulfur are the possible hetero atoms, and is typically an unsaturated heterocyclic radical with five to 18 atoms having at least six conjugated π-electrons such as thienyl, benzo[b]thienyl, dibenzo[b,d]thienyl, thianthrenyl, furyl, furfuryl, 2H-pyranyl, benzofuranyl, isobenzofuranyl, 2H-chromenyl, xanthenyl, dibenzofuranyl, phenoxythienyl, pyrrolyl, imidazolyl, pyrazolyl, pyridyl, bipyridyl, triazinyl, pyrimidinyl, pyrazinyl, 1H-pyrrolizinyl, isoindolyl, pyridazinyl, indolizinyl, isoindolyl, indolyl, 3H- indolyl, phthalazinyl, naphthyridinyl, quinoxalinyl, quinazolinyl, cinnolinyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, 4aH-carbazolyl, carbolinyl, benzotriazolyl, benzoxazolyl, phenanthridinyl, acridinyl, perimidinyl, phenanthrolinyl, phenazinyl, isothiazolyl, phenothiazinyl, isoxazolyl, furazanyl or phenoxazinyl, preferably the above-mentioned mono- or bicyclic heterocyclic radicals, which may be unsubstituted or substituted.

Halogen is fluorine, chlorine, bromine and iodine.

Examples of a five or six membered ring formed by R⁵ and R⁶ are heterocycloalkanes or heterocycloalkenes having from 3 to 5 carbon atoms which can have one additional hetero atom selected from nitrogen, oxygen and sulfur, for example 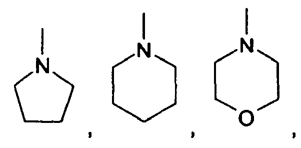 or 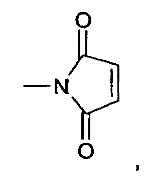 which can be part of a bicyclic system, for example 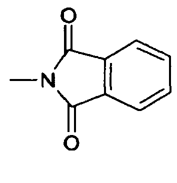 or

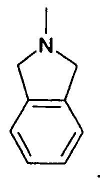

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, a cyano group, an aldehyde group, a ketone group, a carboxyl group, an ester group, a carbamoyl group, an amino group, a nitro group or a silyl group.

As described above, the aforementioned radicals may be substituted by E and/or, if desired, interrupted by D. Interruptions are of course possible only in the case of radicals containing at least 2 carbon atoms connected to one another by single bonds; C₆-C₁₈aryl is not interrupted; interrupted arylalkyl or alkylaryl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR^{z}. CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

The electroluminescent devices may be employed for full color display panels in, for example, mobile phones, televisions and personal computer screens.

In general, the pyrazine compound or compounds emit light below about 520 nm, in particular between about 380 nm and about 520 nm.

The pyrazine compound or compounds have a NTSC coordinate of between about (0.12, 0.05) and about (0.16, 0.10), preferably a NTSC coordinate of about (0.14, 0.08).

The pyrazine compound or compounds have a melting point above about 150°C, preferably above about 200°C and most preferred above about 250°C.

To obtain organic layers of this invention with the proper T_{g}, or glass transition temperature, it is advantageous that the present organic compounds have a melting point greater than about 150°C, for example greater than about 200°C, for example greater than about 250°C, for instance greater than about 300°C.

The electroluminescent devices of the present invention are otherwise designed as is known in the art, for example as described in U.S. Pat. Nos. 5,518,824, 6,225,467, 6,280,859, 5,629,389, 5,486,406, 5,104,740, 5,116,708 and 6,057,048, the relevant disclosures of which are hereby incorporated by reference.

For example, organic EL devices contain one or more layers such as: substrate; base electrode; hole-injecting layer; hole transporting layer; emitter layer; electron-transporting layer; electron-injecting layer; top electrode; contacts and encapsulation.

This structure is a general case and may have additional layers or may be simplified by omitting layers so that one layer performs a plurality of tasks. For instance, the simplest organic EL device consists of two electrodes which sandwich an organic layer that performs all functions, including the function of light emission.

A preferred EL device comprises in this order:
(a) an anode,
(b) a hole injecting layer and/or a hole transporting layer,
(c) a light-emitting layer,
(d) optionally an electron transporting layer and
(e) a cathode.

In particular, the present organic compounds function as light emitters and are contained in the light emission layer or form the light-emitting layer.
The light emitting compounds of this invention exhibit intense fluorescence in the solid state and have excellent electric-field-applied light emission characteristics. Further, the light emitting compounds of this invention are excellent in the injection of holes from a metal electrode and the transportation of holes; as well as being excellent in the injection of electrons from a metal electrode and the transportation of electrons. They are effectively used as light emitting materials and may be used in combination with other hole transporting materials, other electron transporting materials or other dopants.
The organic compounds of the present invention form uniform thin films. The light emitting layers may therefore be formed of the present organic compounds alone.
Alternatively, the light-emitting layer may contain a known light-emitting material, a known dopant, a known hole transporting material or a known electron transporting material as required. In the organic EL device, a decrease in the brightness and life caused by quenching can be prevented by forming it as a multi-layered structure. The light-emitting material, a dopant, a hole-injecting material and an electron-injecting material may be used in combination as required. Further, a dopant can improve the light emission brightness and the light emission efficiency, and can attain the red or blue light emission. Further, each of the hole transporting zone, the light-emitting layer and the electron transporting zone may have the layer structure of at least two layers. In the hole transporting zone in this case, a layer to which holes are injected from an electrode is called "hole-injecting layer", and a layer which receives holes from the hole-injecting layer and transport the holes to a light-emitting layer is called "hole transporting layer". In the electron transporting zone, a layer to which electrons are injected from an electrode is called "electron-injecting layer", and a layer which receives electrons from the electron-injecting layer and transports the electrons to a light-emitting layer is called "electron transporting layer". These layers are selected and used depending upon factors such as the energy level and heat resistance of materials and adhesion to an organic layer or metal electrode.
The light-emitting material or the dopant which may be used in the light-emitting layer together with the organic compounds of the present invention includes for example anthracene, naphthalene, phenanthrene, pyrene, tetracene, coronene, chrysene, fluorescein, perylene, phthaloperylene, naphthaloperylene, perinone, phthaoperinone, naphthaloperinone, diphenylbutadiene, tetraphenylbutadiene, coumarine, oxadiazole, aldazine, bisbenzoxazoline, bisstyryl, pyrazine, cyclopentadiene, quinoline metal complex, aminoquinoline metal complex, benzoquinoline metal complex, imine, diphenylethylene, vinyl anthracene, diaminocarbazole, pyran, thiopyran, polymethine, merocyanine, an imidazole-chelated oxynoid compound, quinacridone, rubrene, and fluorescent dyestuffs for a dyestuff laser or for brightening.
The pyrazine compounds of the present invention and the above compound or compounds that can be used in a light-emitting layer may be used in any mixing ratio for forming a light-emitting layer. That is, the organic compounds of the present invention may provide a main component for forming a light-emitting layer, or they may be a doping material in another main material, depending upon a combination of the above compounds with the organic compounds of the present invention.
The hole-injecting material is selected from compounds which are capable of transporting holes, are capable of receiving holes from the anode, have an excellent effect of injecting holes to a light-emitting layer or a light-emitting material, prevent the movement of excitons generated in a light-emitting layer to an electron-injecting zone or an electron-injecting material and have the excellent capability of forming a thin film. Suitable hole-injecting materials include for example a phthalocyanine derivative, a naphthalocyanine derivative, a porphyrin derivative, oxazole, oxadiazole, triazole, imidazole, imidazolone, imidazolthione, pyrazoline, pyrazolone, tetrahydroimidazole, oxazole, oxadiazole, hydrazone, acylhydrazone, polyarylalkane, stilbene, butadiene, benzidine type triphenylamine, styrylamine type triphenylamine, diamine type triphenylamine, derivatives of these, and polymer materials such as polyvinylcarbazole, polysilane and an electroconducting polymer.
In the organic EL device of the present invention, the hole-injecting material which is more effective is an aromatic tertiary amine derivative or a phthalocyanine derivative. Although not specially limited, specific examples of the tertiary amine derivative include triphenylamine, tritolylamine, tolyldiphenylamine, N,N'-diphenyl-N,N'-(3-methylphenyl)-1,1-biphenyl-4,4'-diamine, N,N,N',N'-tetra(4-methylphenyl)-1,1'-phenyl-4,4'-diamine, N,N,N',N'-tetra(4-methylphenyl)-1,1'-biphenyl-4,4'-diamine, N,N'-diphenyl-N,N'-di(1-naphthyl)-1,1'-biphenyl-4,4'-diamine, N,N'-di(methylphenyl)-N,N'-di(4-n-butylphenyl)-phenanthrene-9,10- diamine, 4,4', 4"-tris(3-methylphenyl)-N-phenylamino)triphenylamine, 1,1-bis(4-di-p-tolylaminophenyl)cyclohexane, and oligomers or polymers having aromatic tertiary amine structures of these.
Although not specially limited, specific examples of the phthalocyanine (Pc) derivative include phthalocyanine derivatives or naphthalocyanine derivatives such as H₂Pc, CuPc, CoPc, NiPc, ZnPc, PdPc, FePc, MnPc, ClAlPc, CIGaPc, ClInPc, ClSnPc, Cl₂SiPc, (HO)AlPc, (HO)GaPc, VOPc, TiOPc, MoOPc, and GaPc-O-GaPc.
The hole transporting layer can reduce the driving voltage of the device and improve the confinement of the injected charge recombination within the pyrazine light emitting layer. Any conventional suitable aromatic amine hole transporting materials described for the hole-injecting layer may be selected for forming this layer.
A preferred class of hole transporting materials is comprised of 4,4'-bis(9-carbazolyl)-1,1'-biphenyl compounds of the formula 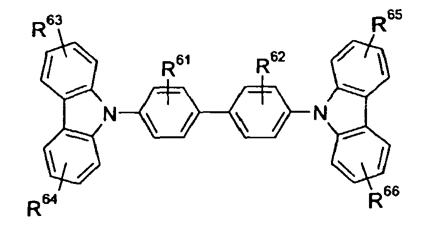 wherein R⁶¹ and R⁶² is a hydrogen atom or a C₁-C₃alkyl group; R⁶³ through R⁶⁶ are substituents independently selected from the group consisting of hydrogen, a C₁-C₆alkyl group, a C₁-C₆alkoxy group, a halogen atom, a dialkylamino group, a C₆-C₃₀aryl group, and the like. Illustrative examples of 4,4'-bis(9-carbazolyl)-1,1'-biphenyl compounds include 4,4'-bis(9-carbazolyl)-1,1'-biphenyl and 4,4'-bis(3-methyl-9-carbazolyl)-1,1'-biphenyl, and the like.
The electron transporting layer is not necessarily required for the present device, but is optionally and preferably used for the primary purpose of improving the electron injection characteristics of the EL devices and the emission uniformity. Illustrative examples of electron transporting compounds, which can be utilized in this layer, include the metal chelates of 8-hydroxyquinoline as disclosed in U.S. Pat. Nos. 4,539,507, 5,151,629, and 5,150,006, the disclosures of which are totally incorporated herein by reference.
Although not specially limited, specific examples of the metal complex compound include lithium 8-hydroxyquinolinate, zinc bis(8-hydroxyquinolinate), copper bis(8-hydroxyquinolinate), manganese bis(8-hydroxyquinolinate), aluminum tris(8-hydroxyquinolinate), aluminum tris(2-methyl-8-hydroxyquinolinate), gallium tris(8-hydroxyquinolinate), beryllium bis(10-hydroxybenzo[h]quinolinate), zinc bis(10-hydroxybenzo[h]quinolinate), chlorogallium bis(2-methyl-8-quinolinate), gallium bis(2-methyl-8-quinolinate)(o-cresolate), aluminum bis(2-methyl-8-quinolinate)(1-naphtholate), gallium bis(2-methyl-8-quinolinate)(2-naphtholate), gallium bis(2-methyl-8-quinolinate)phenolate, zinc bis(o-(2-benzooxazolyl)phenolate), zinc bis(o-(2-benzothiazolyl)phenolate) and zinc bis(o-(2-benzotrizolyl)phenolate). The nitrogen-containing five-membered derivative is preferably an oxazole, thiazole, thiadiazole, or triazole derivative. Although not specially limited, specific examples of the above nitrogen-containing five-membered derivative include 2,5-bis(1-phenyl)-1,3,4-oxazole, 1,4-bis(2-(4-methyl-5-phenyloxazolyl)benzene, 2,5-bis(1-phenyl)-1,3,4-thiazole, 2,5-bis(1-phenyl)-1,3,4-oxadiazole, 2-(4'-tert-butylphenyl)-5-(4"-biphenyl)1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4"-biphenyl)-1,3,4-thiadiazole, 2,5-bis(1-naphthyl)-1,3,4-thiadiazole, 1,4-bis[2-(5-phenylthiazolyl)]benzene, 2-(4'-tert-butylphenyl)-5-(4"-biphenyl)-1,3,4-triazole, 2,5-bis(1-naphthyl)-1,3,4-triazole and 1,4-bis[2-(5-phenyltriazolyl)]benzene. Another class of electron transport materials are oxadiazole metal chelates, such as bis[2-(2-hydroxyphenyl)-5-phenyl-1,3,4-oxadiazolato]zinc; bis[2-(2-hydroxyphenyl)-5-phenyl-1,3,4-oxadiazolato]beryllium; bis[2-(2-hydroxyphenyl)-5-(1-naphthyl)-1,3,4-oxadiazolato]zinc; bis[2-(2-hydroxyphenyl)-5-(1-naphthyl)-1,3,4-oxadiazolato]beryllium; bis[5-biphenyl-2-(2-hydroxyphenyl)-1,3,4-oxadiazolato]zinc; bis[5-biphenyl-2-(2-hydroxyphenyl)-1,3,4-oxadiazolato]beryllium; bis(2-hydroxyphenyl)-5-phenyl-1,3,4-oxadiazolato]lithium; bis[2-(2-hydroxyphenyl)-5-p-tolyl-1,3,4-oxadiazolato]zinc; bis 2-(2-hydroxyphenyl)-5-p-tolyl-1,3,4-oxadiazolato]beryllium; bis[5-(p-tert-butylphenyl)-2-(2-hydroxyphenyl)-1,3,4-oxadiazolato]zinc; bis[5-(p-tert-butylphenyl)-2-(2-hydroxyphenyl)-1,3,4-oxadiazolato]berylliu m; bis[2-(2-hydroxyphenyl)-5-(3-fluorophenyl)-1,3,4-0xadiazolato]zinc; bis[2-(2-hydroxyphenyl)-5-(4-fluorophenyl)-1,3,4-oxadiazolato]zinc; bis[2-(2-hydroxyphenyl)-5-(4-fluorophenyl)-1,3,4-oxadiazolato]beryllium; bis[5-(4-chlorophenyl)-2-(2-hydroxyphenyl)-1,3,4-oxadiazolato]zinc; bis[2-(2-hydroxy phenylr5-(4-methoxyphenyl)-1,3,4-oxadiazolato]zinc; bis[2-(2-hydroxy-4-rnethylphenyl)-5-phenyl-1,3,4-oxadiazolato]zinc; bis[2-.alpha.-(2-hydroxynaphthyl)-5-phenyl-1,3,4-oxadiazolato]zinc; bis[2-(2-hydroxyphenyl)-5-p-pyridyl-1,3,4-oxadiazolato]zinc; bis[2-(2-hydroxyphenyl)-5-p-pyridyl-1,3,4-oxadiazolato]beryllium; bis[2-(2-hydroxyphenyl)-5-(2-thiophenyl)-1,3,4-oxadiazolato]zinc; bis[2-(2-hydroxyphenyl)-5-phenyl-1,3,4-thiadiazolato]zinc; bis[2-(2-hydroxyphenyl)-5-phenyl-1,3,4-thiadiazolato]beryllium; bis[2-(2-hydroxyphenyl)-5-(1-naphthyl)-1,3,4-thiadiazolatoJzinc; and bis[2-(2-hydroxyphenyl)-5-(1-naphthyl)-1,3,4-thiadiazolato]beryllium, and the like.

In the organic EL device of the present invention, the light-emitting layer may contain, in addition to the light-emitting organic material of the present invention, at least one of other light-emitting material, other dopant, other hole-injecting material and other electron-injecting material. For improving the organic EL device of the present invention in the stability against temperature, humidity and ambient atmosphere, a protective layer may be formed on the surface of the device, or the device as a whole may be sealed with a silicone oil, or the like.
The electrically conductive material used for the anode of the organic EL device is suitably selected from those materials having a work function of greater than 4 eV. The electrically conductive material includes carbon, aluminum, vanadium, iron, cobalt, nickel, tungsten, silver, gold, platinum, palladium, alloys of these, metal oxides such as tin oxide and indium oxide used for ITO substrates or NESA substrates, and organic electroconducting polymers such as polythiophene and polypyrrole.
The electrically conductive material used for the cathode is suitably selected from those having a work function of smaller than 4 eV. The electrically conductive material includes magnesium, calcium, tin, lead, titanium, yttrium, lithium, ruthenium, manganese, aluminum and alloys of these, while the electrically conductive material shall not be limited to these. Examples of the alloys include magnesium/silver, magnesium/indium and lithium/aluminum, while the alloys shall not be limited to these. Each of the anode and the cathode may have a layer structure formed of two layers or more as required.
For the effective light emission of the organic EL device, at least one of the electrodes is desirably sufficiently transparent in the light emission wavelength region of the device. Further, the substrate is desirably transparent as well. The transparent electrode is produced from the above electrically conductive material by a deposition method or a sputtering method such that a predetermined light transmittance is secured. The electrode on the light emission surface side has for instance a light transmittance of at least 10%. The substrate is not specially limited so long as it has adequate mechanical and thermal strength and has transparency. For example, it is selected from glass substrates and substrates of transparent resins such as a polyethylene substrate, a polyethylene terephthalate substrate, a polyether sulfone substrate and a polypropylene substrate.
In the organic EL device of the present invention, each layer can be formed by any one of dry film forming methods such as a vacuum deposition method, a sputtering method, a plasma method and an ion plating method and wet film forming methods such as a spin coating method, a dipping method and a flow coating method. The thickness of each layer is not specially limited, while each layer is required to have a proper thickness. When the layer thickness is too large, inefficiently, a high voltage is required to achieve predetermined emission of light. When the layer thickness is too small, the layer is liable to have a pinhole, etc., so that sufficient light emission brightness is hard to obtain when an electric field is applied. The thickness of each layer is for example in the range of from about 5 nm to about 10 µm, for instance about 10 nm to about 0.2 µm.
In the wet film forming method, a material for forming an intended layer is dissolved or dispersed in a proper solvent such as ethanol, chloroform, tetrahydrofuran and dioxane, and a thin film is formed from the solution or dispersion. The solvent shall not be limited to the above solvents. For improving the film formability and preventing the occurrence of pinholes in any layer, the above solution or dispersion for forming the layer may contain a proper resin and a proper additive. The resin that can be used includes insulating resins such as polystyrene, polycarbonate, polyarylate, polyester, polyamide, polyurethane, polysulfone, polymethyl methacrylate, polymethyl acrylate and cellulose, copolymers of these, photoconductive resins such as poly-N-vinylcarbozole and polysilane, and electroconducting polymers such as polythiophene and polypyrrole. The above additive includes an antioxidant, an ultraviolet absorbent and a plasticizer.
When the light-emitting organic material of the present invention is used in a light-emitting layer of an organic EL device, an organic EL device can be improved in organic EL device characteristics such as light emission efficiency and maximum light emission brightness. Further, the organic EL device of the present invention is remarkably stable against heat and electric current and gives a usable light emission brightness at a low actuation voltage. The problematic deterioration of conventional devices can be remarkably decreased.
The organic EL device of the present invention has significant industrial values since it can be adapted for a flat panel display of an on-wall television set, a flat light-emitting device, a light source for a copying machine or a printer, a light source for a liquid crystal display or counter, a display signboard and a signal light.

The material of the present invention can be used in the fields of an organic EL device, an electrophotographic photoreceptor, a photoelectric converter, a solar cell, an image sensor, dye lasers and the like.

The following Examples illustrate the invention, without limiting the scope thereof. In the Examples and throughout this application, the term light emitting material means the present pyrazine compounds.

### Examples

### Example 1

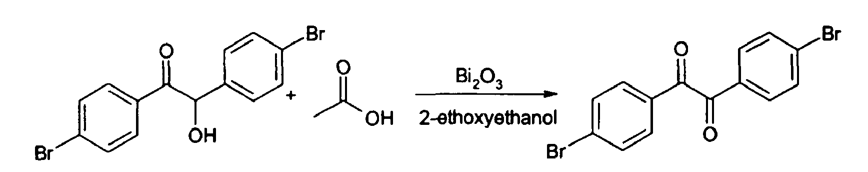

a) 1,2-Di(4-bromophenyl)-2-hydroxyethanone (0.50 g, 1.4 mmol) is added to 20 ml of 2-ethoxyethanol and 0.3 ml of AcOH. The mixture is heated to 105°C and then Bi₂O₃ (0.19 g, 0.4 mmol) is added. CH₂Cl₂ is added to the reaction mixture and an extraction is made with water. The organic phase is washed until the water phase is neutral. The product is redissolved in toluene and filtrated on silica gel. The solvent is evaporated to leave the product as a yellow crystalline material (yield: 0.37 g, 72%, mp. 225-227°C). ¹H NMR (300 MHz, CDCl₃): δ 7.77 (d, 10.8 Hz, 4H), 7.60 (d, 10.8 Hz, 4H). 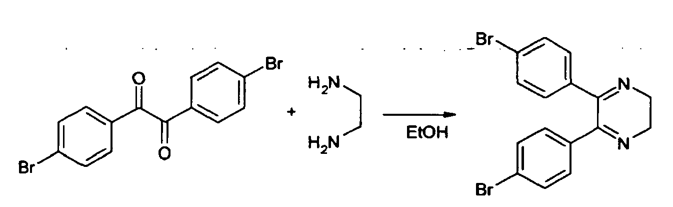
b) First the product obtained in step a) (10.01 g, 27 mmol) is added to 100 ml of ethanol, then ethylene diamine (1.99 g, 33 mmol) is added and the reaction mixture is refluxed for 2 h. During cooling a product precipitates, which is filtered and dried to give a yellow crystalline material (yield: 9.80 g, 92 %). ¹H NMR (300 MHz, CDCl₃): δ 7.43 (d, 4.7 Hz, 4H), 7.27 (d, 4.8 Hz, 4H), 3.70 (s, 2H). 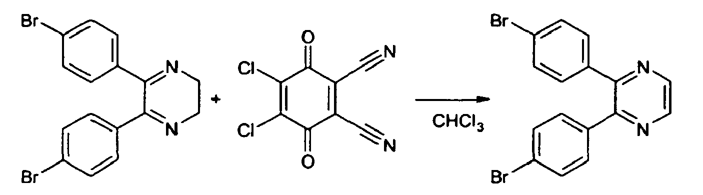
c) The dihydropyrazine (9.02 g, 23 mmol) is dissolved in 50 ml of chloroform and then DDQ (10.44 g, 46 mmol) is added. The reaction mixture is refluxed for 8 h, poured into a NaHCO₃ solution and the water phase is extracted with dichloromethane. The organic phase is washed with NaHCO₃ until the water phase is nearly colourless. The product is purified by column chromatography with dichloromethane as eluant. After evaporation, a white solid is obtained (yield: 7.81 g, 87 %; mp 153-154 °C). ¹H NMR (300 MHz, CDCl₃): δ 8.53 (s, 2H), 7.39 (d, 6.7 Hz, 4H), 7.26 (d, 6.7 Hz, 4H).

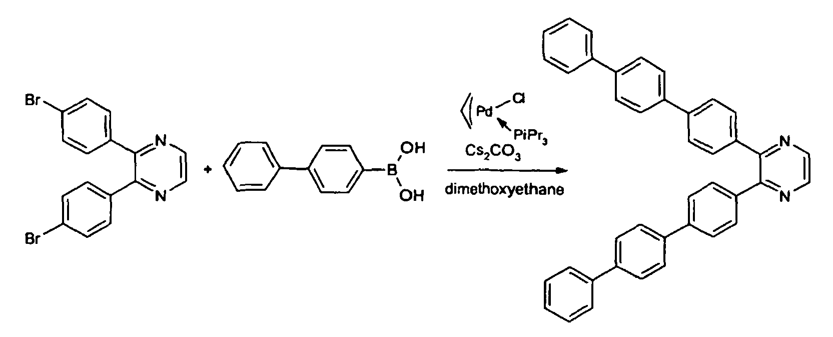

The product obtained in step c) (0.98 g, 2.5 mmol) is added to 50 ml of dimethoxyethane, then biphenylboronic acid (1.24 g, 6.3 mmol) is added and the reaction mixture is stirred under Argon atmosphere for 10 minutes. Cs₂CO₃ (2.04 g, 6.3 mmol) dissolved in 5 ml of water is added. Then the palladium catalysator is added. The reaction mixture is refluxed for 18h. The product is filtered off and recrystallized in DMF to give a grey crystalline material (yield: 0.92 g, 69 %, mp 298-301°C). ¹H NMR (400 MHz, CDCl₃): δ 8.69 (s, 2H), 7.77-7.68 (m, 20H), 7.51 (t, 7.6 Hz, 4H), 7.43-7.39 (m, 2H).

### Example 2

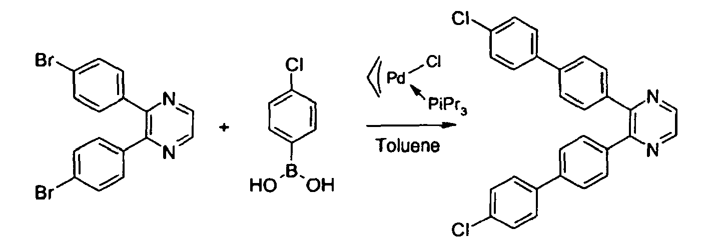

The product obtained in step c) of example 1 (2.00 g, 5.1 mmol) and 4-chlorophenylboronic acid (2.41 g, 15.4 mmol) are added to 100 ml of toluene. The mixture is stirred for 10 minutes under an argon atmosphere. Then Cs₂CO₃ (7.86 g, 24.1 mmol) in 4 ml of water is slowly added to the mixture. After 10 minutes the palladium catalysator is added. The reaction mixture is refluxed for 7 h, CH₂Cl₂ is added and the solution is extracted with a saturated solution of tartaric acid. The product is recrystallized in ethanol to give a white crystalline material (yield: 1.94 g, 84 %, mp 184-185°C). ¹H NMR (300 MHz, CDCl₃): δ 8.56 (s, 2H), 7.52 (d, 6.6 Hz, 8H), 7.46 (d, 7.8 Hz, 4H), 7.33 (d, 7.0 Hz, 4H).

### Example 3

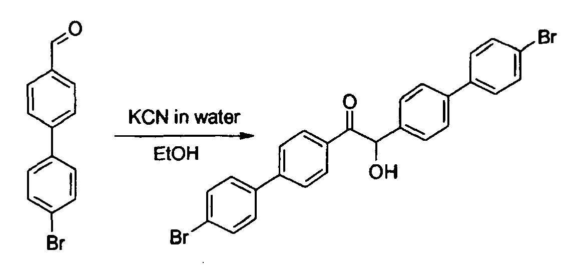

a) 4-(4'-Bromobiphenyl)methanal (4.18 g, 16 mmol) is added to 10 ml of ethanol. Then KCN (0.03 g, 0.5 mmol) in 5 ml of water is added. The reaction mixture is refluxed. After 90 minutes KCN is added. After 4 h the reaction is finished. The solid is filtered, washed with ethanol, H₂O and ethanol to give a pale yellow solid (yield: 3.39 g (81%), mp. 243-246°C). ¹H NMR (400 MHz, CDCl₃): δ 8.29 (d, 6.7 Hz, 2H), 7.87-7.78 (m, 8H), 7.70-7.64 (m, 6H), 6.27 (d, 6.0 Hz, 1 H), 4.84 (d, 6.1 Hz, 1H). 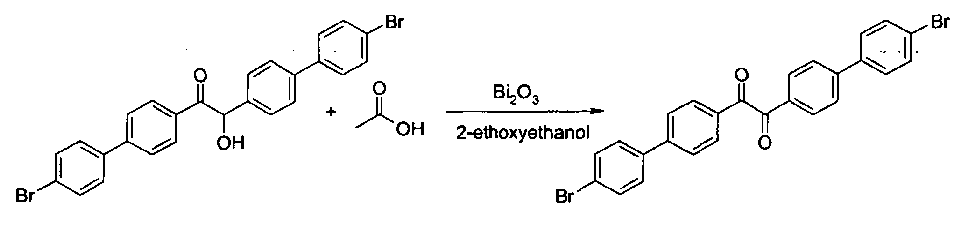
b) The product obtained in step a) (0.80 g, 1.5 mmol) is added to 40 ml of 2-ethoxyethanol and 0.5 ml of AcOH. The mixture is heated at 105°C and then Bi₂O₃ (0.19 g, 0.4 mmol) is added. After 3 h the reaction is finished. The grey-green product is filtered off (yield: 0.70 g, 88 %, mp 259.5-260.5°C). ¹H NMR (400 MHz, CDCl₃): δ 8.23 (d, 8.0 Hz, 4H), 7.86 (d, 8.4 Hz, 4H), 7.77 (d, 6.8 Hz, 4H), 7.65 (d, 6.8 Hz, 4H). 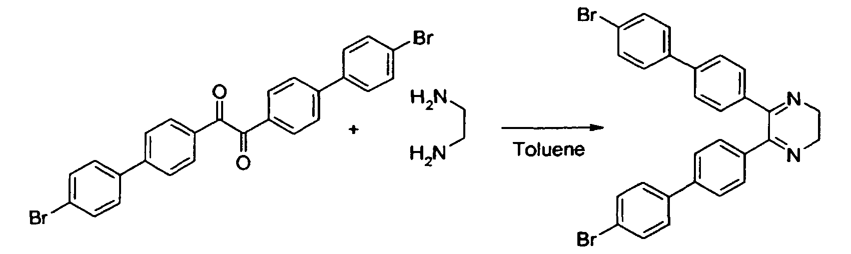
c) the product obtained in step b) (1.80 g, 3.5 mmol) is added to 50 ml of toluene, then ethylene diamine (0.42 g, 6.9 mmol) is added and the reaction mixture is refluxed for 4 h. During cooling a grey crystalline material precipitates, which was filtered off and dried (yield: 1.43 g (76 %). ¹H NMR (400 MHz, CDCl₃): δ 7.60-7.44 (m, 16H), 3.76 (s, 4H). 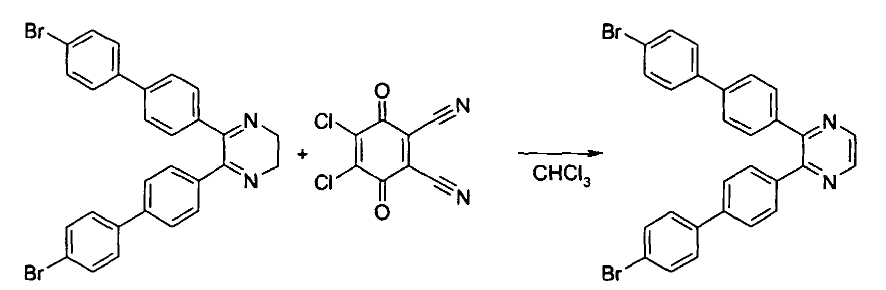
d) The product obtained in step c) (0.33 g, 0.6 mmol) is added to 10 ml of chloroform, and then DDQ (0.27 g, 1.2 mmol) is added. The reaction mixture is refluxed for 4 h. The reaction mixture is poured in a NaHCO₃ solution and the water phase is extracted with dichloromethane. The organic phase is washed with NaHCO₃ until the water phase is nearly colourless. The solvent is removed in vacuum to give a brown-orange solid (yield: 0.3 g (92 %), mp 214-214.5°C). ¹H NMR (300 MHz, CDCl₃): δ 8.56 (s, 2H), 7.54-7.38 (m, 16H). 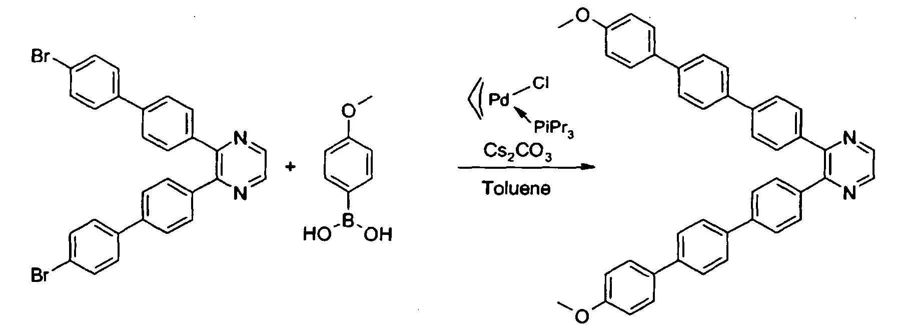
e) The product obtained in step d) (0.80 g, 1.5 mmol) and 4-methoxyphenylboronic acid (0.56 g, 3.7 mmol) are added to 40 ml of toluene. The mixture is stirred for 10 min under an argon atmosphere. Then Cs₂CO₃ (1.41 g, 4.3 mmol) in 5 ml of water is slowly added to the mixture. After 10 minutes palladium catalysator is added. Then the reaction mixture is refluxed for 15 h. The solid phase is filtered off and washed. The product is recrystallized in DMF and then filtered on Hyflo. After solvent evaporation a pale yellow solid remains (yield: 0.20 g (23 %, mp. 339.5-341°C). ¹H NMR (400 MHz, CDCl₃): δ 8.66 (s, 2H), 7.71-7.59 (m, 20H), 7.01 (d, 8.8 Hz, 4H), 3.88 (s, 6H).

### Example 4

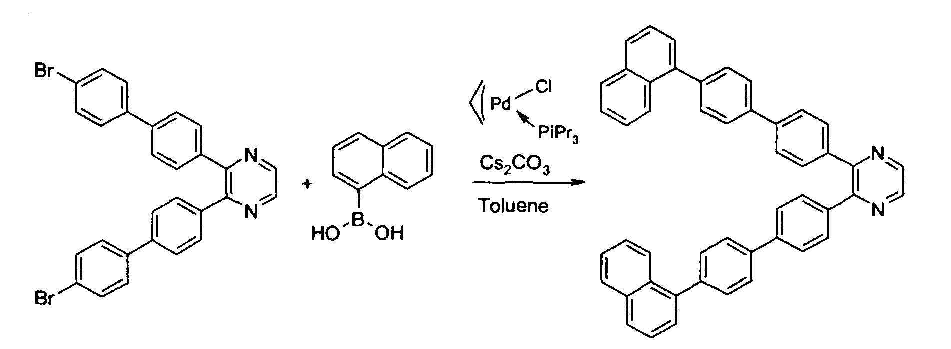

The product obtained in example 3b) (1.20 g, 2.7 mmol) and 1-naphtylboronic acid (1.14 g, 6.6 mmol) are added to 50 ml of toluene. The mixture is stirred for 10 minutes under an argon atmosphere. Then Cs₂CO₃ (2.54 g, 7.8 mmol) in 8 ml of water and the palladium catalysator are added. The reaction mixture is refluxed for 18 h. Then the solution is poured into 10 % tartaric acid and an extraction is made with dichloromethane. The organic phase is dried with magnesium sulphate and the solvent is removed. Then the crude product was purified by column chromatography on silica gel with dichloromethane. After evaporation a white powder is recovered. Yield: 1.43 g (2.25 mmol) 85%. ¹H NMR (400 MHz, CDCl₃): δ 8.69 (s, 2H), 8.00-7.89 (m, 6H), 7.79 (d, 8.2 Hz, 4H), 7.74-7.72 (m, 8H), 7.69-7.45 (m, 12H).

### Example 5

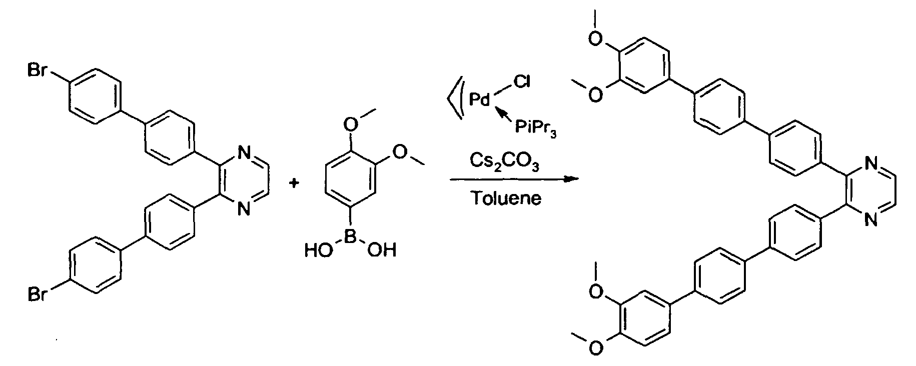

The product obtained in example 3b) (1.20 g, 2.7 mmol) and 3,4-dimethoxyphenylboronic acid (1.20 g, 6.6 mmol) are added to 50 ml of toluene. The mixture is stirred for 10 minutes under an argon atmosphere. Then Cs₂CO₃ (2.54 g, 7.8 mmol) in 8 ml of water and the palladium catalysator are added. The reaction mixture is refluxed. After 12 h one equivalent of each reactant is added. The reaction is finished after 18 h. The reaction mixture is then poured into 10 % tartaric acid and an extraction is made with dichloromethane. The organic phase is dried with magnesium sulphate and the solvent is removed. The crude product is then dissolved in CH₂Cl₂ and filtered, wherein a gold crystalline product is obtained (yield: 0.64 g (37%). ¹H NMR (400 MHz, CDCl₃): δ 8.66 (s, 2H), 7.71 (d, 8.4 Hz, 4H), 7.66 (m, 12H), 7.22 (dd, 1.9, 8.3 Hz, 2H), 7.17 (d, 1.9 Hz, 2H), 3.99 (s, 6H), 3.96 (s, 6H).

### Example 6

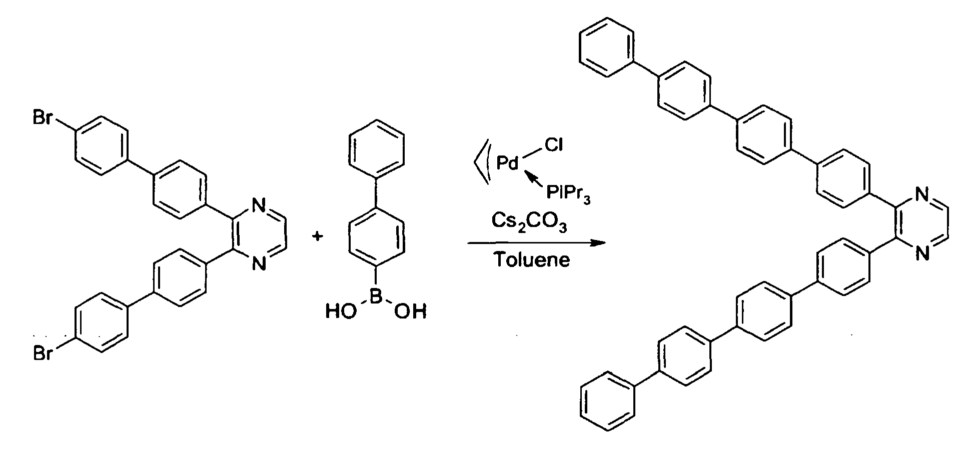

The product obtained in example 4b) (1.40 g, 2.6 mmol) and biphenylboronic acid (1.28 g, 6.5 mmol) are put in 60 ml of toluene. The mixture is stirred for 10 minutes under an argon atmosphere. Then Cs₂CO₃ in 10 ml of water is slowly added to the mixture. After 10 minutes the palladium catalyxsator is added. Then the reaction mixture is refluxed for 72 h. The solid phase is filtered off. The product is recrystallised from isopropanol to obtain a brown solid (yield: 0.20 g (11 %)). ¹H NMR (400 MHz, CDCl₃): δ 8.66 (s, 2H), 7.80-4.63 (m, 26H), 7.54-7.36 (m, 8H).

### Application Example (Device)

The following device structure is prepared: ITO/CuPC/TCTA/ Compound of Example 4/TPBI/LiF/Al where ITO is indium tin oxide, CuPC is copper phthalocyanine, TCTA is 4,4',4"-tri-(N-carbazoyl)triphenylamine, and TPBI is 1,3,5-tris-(N-phenyl-benzimidazol-2-yl) benzene. Using this device structure, a brightness of 106 cd/m² is observed with a efficiency of 0.39 cd/A at 11 V with an emission λₘₐₓ at 450 nm.

## Claims

1. A pyrazine compound of formula 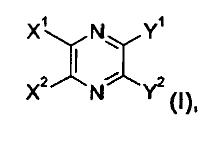 wherein
X¹ is a group of formula 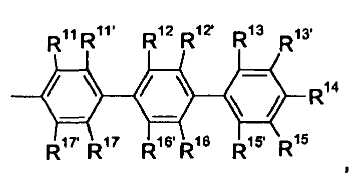 or a C₁₆-C₃₀aryl group, which can optionally be substituted by E;
X² is an aryl group, or a heteroaryl group, which can optionally be substituted; especially a group of formula 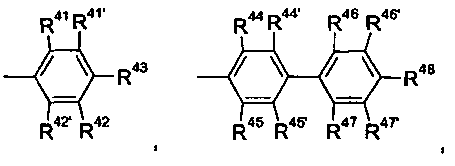 or 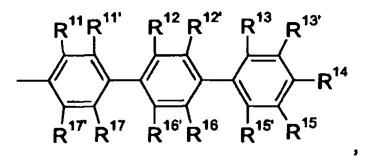 or a C₁₆-C₃₀aryl group, which can optionally be substituted by E;
Y¹ and Y² are independently of each other a hydrogen atom, C₁-C₁₈alkyl, which is optionally interrupted by O,
an aryl group or a heteroaryl group, which can optionally be substituted; especially a C₁₆-C₃₀aryl group, which can optionally be substituted by E; or a group of formula 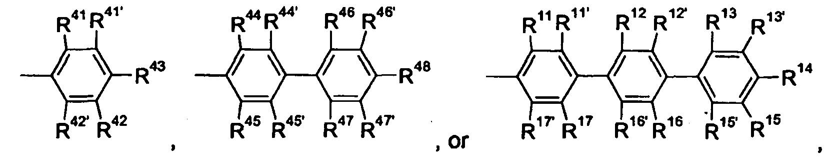 or
Y¹ and Y² together form a C₅-C₈cycloalkyl group, wherein
R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ and R^{47'} are independently of each other H, E, C₆-C₁₈aryl; C₆-C₁₈aryl which is substituted by E; C₁-C₁₈alkyl; C₁-C₁₈alkyl which is substituted by E' and/or interrupted by D; C₇-C₁₈aralkyl; or C₇-C₁₈aralkyl which is substituted by E; or
R^{11'} and R¹², R^{12'} and R¹³, R^{15'} and R¹⁶, R^{16'} and R¹⁷, R^{44'} and R⁴⁶ and/or R^{45'} and R⁴⁷ are each a divalent group L¹ selected from an oxygen atom, an sulfur atom, >CR¹⁸R¹⁹ >SiR¹⁸R¹⁹, or 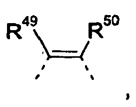 wherein
R¹⁸ and R¹⁹ are independently of each other C₁-C₁₈alkyl; C₁-C₁₈alkoxy, C₆-C₁₈aryl, C₆-C₁₈aryl, which is substituted by E; C₇-C₁₈aralkyl, or C₇-C₁₈aralkyl, which is substituted by E; or
R¹¹ and R^{11'}, R¹² and R^{12'}, R¹³ and R^{13'}, R^{13'} and R¹⁴, R¹⁴ and R¹⁵, R¹⁵ and R^{15'}, R¹⁶ and R^{16'}, R^{17'} and R¹⁷, R⁴¹ and R^{41'}, R⁴² and R^{42'}, R^{42'} and R⁴³, R^{41'} and R⁴³, R⁴⁴ and R^{44'}, R⁴⁵ and R^{45'}, R⁴⁶ and R^{46'}, R⁴⁷ and R^{47'}, R^{46'} and R⁴⁸ and/or R^{47'} and R⁴⁸ are each a divalent group 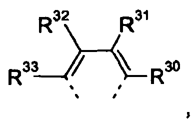 wherein
R³⁰, R³¹, R³², R³³, R⁴⁹ and R⁵⁰ are independently of each other H, C₁-C₁₈alkyl; C₁-C₁₈alkyl, which is substituted by E' and/or interrupted by D; E; C₆-C₁₈aryl; C₆-C₁₈aryl, which is substituted by E;
R¹⁴ is H, C₂-C₃₀heteroaryl, -NR⁷⁰R⁷¹, C₆-C₃₀aryl, or C₆-C₃₀aryl which is substituted by E, C₁-C₁₈alkyl; or C₁-C₁₈alkyl which is substituted by E' and/or interrupted by D; especially 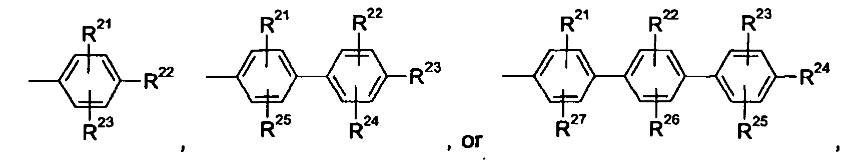 wherein
R²¹, R²², R²³, R²⁴, R²⁵, R²⁶ and R²⁷ are independently of each other H, E, C₁-C₁₈alkyl; C₁-C₁₈alkyl which is substituted by E' and/or interrupted by D; E; C₇-C₁₈aralkyl; C₇-C₁₈aralkyl which is substituted by E;
R⁴³ and R⁴⁸ are independently of each other H, E; especially C₁-C₂₄alkyl, C₁-C₂₄alkoxy, or -NR⁷⁰R⁷¹, wherein R⁷⁰ and R⁷¹ are independently of each other H, C₈-C₁₈aryl, C₆-C₁₈aryl which is substituted by C₁-C₂₄alkyl, or C₁-C₂₄alkoxy; C₁-C₂₄alkyl, or C₁-C₂₄alkyl which is interrupted by -O-, or
R⁷⁰ and R⁷¹ together form a five or six membered ring, in particular 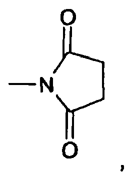 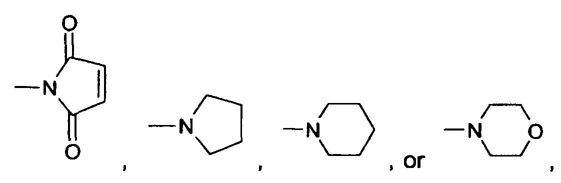 C₁-C₁₈alkyl; C₁-C₁₈alkyl, which is substituted by E and/or interrupted by D; C₂-C₃₀heteroaryl; C₇-C₁₈aralkyl; C₇-C₁₈aralkyl which is substituted by E;
D is -CO-; -COO-; -OCOO-; -S-; -SO-; -SO₂-; -O-; -NR⁵-; -SiR⁶¹R⁶²-; -POR⁵-; -CR⁶³=CR⁶⁴-; or -C≡C-;
E is C₁-C₁₈alkyl, -OR⁵; -SR⁵; -NR⁵R⁶; -COR⁸; -COOR⁷; -CONR⁵R⁶; -CN; or halogen;
E' is E, except C₁-C₁₈alkyl, wherein
R⁵ and R⁶ are independently of each other C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -O-; or
R⁵ and R⁶ together form a five or six membered ring, in particular 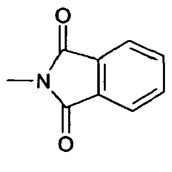 or 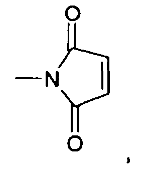
R⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-;
R⁸ is C₇-C₁₂alkylaryl; C₁-C₁₈alkyl; or C₁-C₁₈alkyl which is interrupted by -0-;
R⁶¹ and R⁶² are independently of each other C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -O-, and
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl, or C₁-C₁₈alkyl which is interrupted by -O-.

2. A pyrazine compound of formula I according to claim 1, wherein X¹ and X² are independently of each other a group of formula 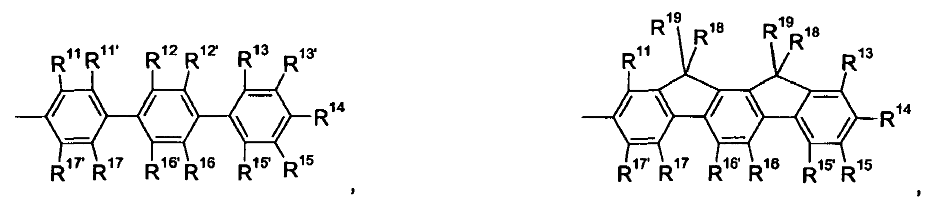 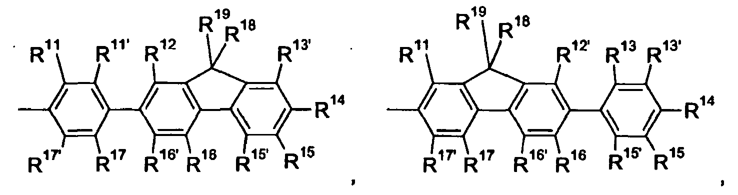 or -X¹¹-X¹²-X¹³,
wherein
R¹¹, R^{11'}, R¹², R^{12'}, R¹³, R^{13'}, R¹⁵, R^{15'}, R¹⁶, R^{16'}, R¹⁷ and R^{17'} are independently of each other H, C₆-C₁₈aryl; C₆-C₁₈aryl which is substituted by E; E, C₁-C₁₈alkyl; C₁-C₁₈alkyl which is substituted by E' and/or interrupted by D; C₇-C₁₈aralkyl; C₇-C₁₈aralkyl which is substituted by E; and
R¹⁴, R¹⁸ and R¹⁹ are as defined in claim 1,
X¹¹ and X¹² are independently of each other a group of formula 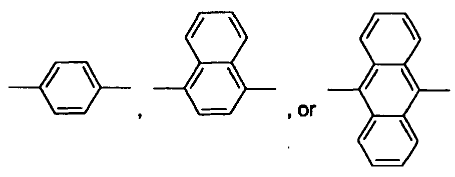 and X¹³ is a group of formula 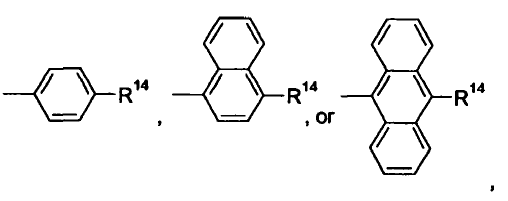 wherein R¹⁴ is 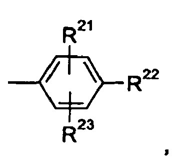 or 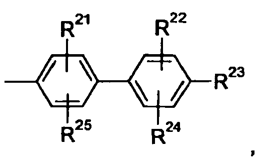 wherein R²¹, R²², R²³, R²⁴ and R²⁵ are as defined in claim 1 and Y¹ and Y² are a hydrogen atom, C₁-C₁₈alkyl, which is optionally interrupted by O, or Y¹ and Y² together form a C₅-C₈cycloalkyl group, and are especially a hydrogen atom.

3. The pyrazine compound according to claim 1 or 2, wherein R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, and R^{47'} as well as R¹⁴, R⁴³, and R⁴⁸ are independently of each other H, E; or C₁-C₈alkyl; wherein E is -OR⁵; -SR⁵; -NR⁵R⁶; -COR⁸; -COOR⁷; -CONR⁵R⁶; -CN; -OCOOR⁷; or halogen; wherein R⁵ and R⁶ are independently of each other C₆-C₁₂aryl, or C₁-C₈alkyl;
R⁷ is C₇-C₁₂alkylaryl, or C₁-C₈alkyl; and
R⁸ is C₆-C₁₂aryl; or C₁-C₈alkyl; or
R¹¹ and R^{11'}, R¹² and R^{12'}, R¹³ and R^{13'}, R^{13'} and R¹⁴, R⁴¹ and R^{41'}, R^{41'} and R⁴³, R⁴⁴ and
R^{44'}, R⁴⁶ and R^{46'}, R^{46'} and R⁴⁸ and/or R^{47'} and R⁴⁸ are each a divalent group

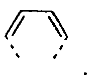

4. The pyrazine compound according to claim 1, wherein
X¹ and X² are a group of formula 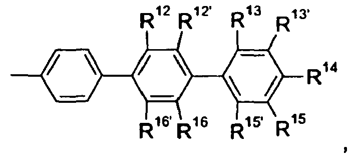 wherein
R¹³, R^{13'}, R¹⁵ and R^{15'} are H and R¹⁴ is H, or 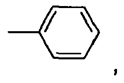 and R¹², R^{12'}, R¹⁶ and R^{16'} are H; or
R¹³ and R¹⁵ are H, R^{13'} and R^{15'} are independently of each other H, C₁-C₈alkyl, or C₁-C₈alkoxy, and R¹⁴ is H, C₁-C₈alkyl, or C₁-C₈alkoxy, and R¹², R^{12'}, R¹⁶ and R^{16'} are H, wherein at least one of R¹³, R¹⁵, R^{13'}, R^{15'} and R¹⁴ is C₁-C₈alkyl, or C₁-C₈alkoxy;
R¹² and R^{12'}, R¹³ and R^{13'}, R^{13'} and R¹⁴, R¹⁴ and R¹⁵, R¹⁵ and R^{15'}, and/or R¹⁶ and R^{16'}, can be a divalent group 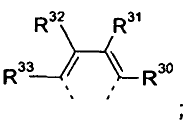 or
R^{12'}, R¹⁶, R^{16'} are H and R¹³ and R^{13'}, or R^{13'} and R¹⁴ and/or R¹⁵ and R^{15'} are a divalent group 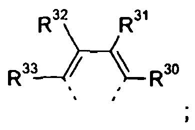 or
R¹³, R^{13'}, R¹⁴, R¹⁵, R^{15'} are H, R¹⁴ is H, C₁-C₈alkyl and R¹² and R^{12'}, and/or R¹⁶ and R^{16'} are a divalent group 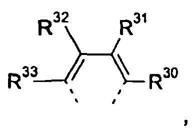 wherein R³⁰, R³¹, R³² and R³³ are H, C₁-C₈alkyl, or C₁-C₈alkoxy, and
Y¹ and Y² are a hydrogen atom.

5. The pyrazine compound according to any of claims 1 to 3, wherein X¹ and X² are independently of each other a group of formula 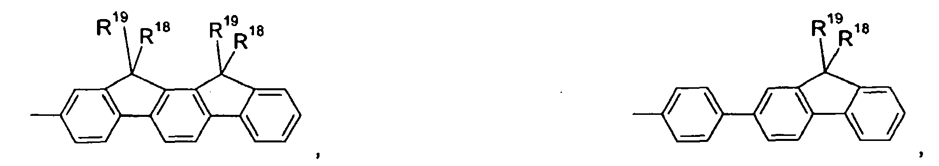 or 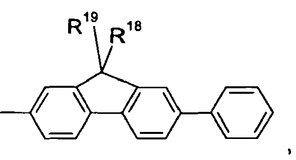 wherein
R¹⁸ and R¹⁹ are independently of each other C₁-C₈alkyl.

6. The pyrazine compound according to claim 1, wherein
X¹ is a group of formula 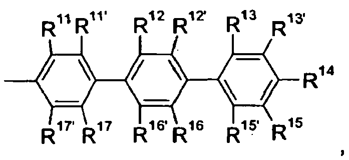 especially 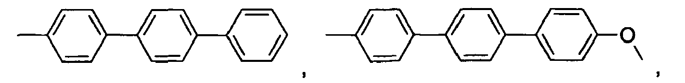 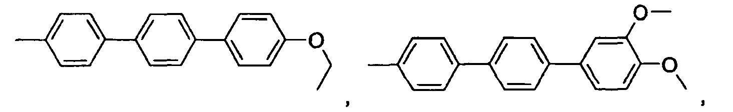 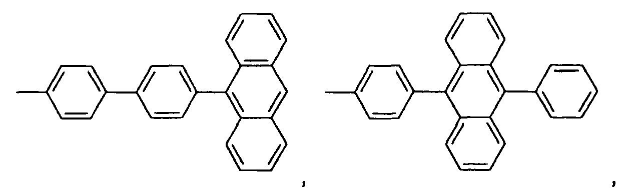 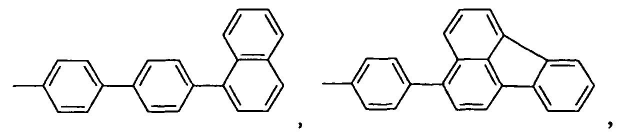 or 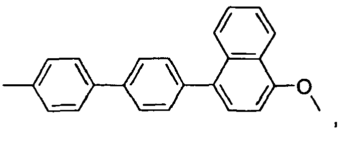 and
X² is a group of formula 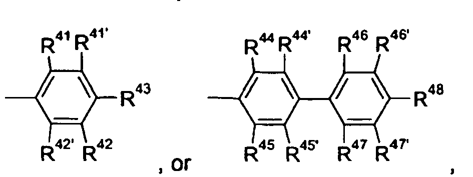 especially 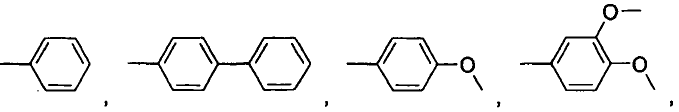 or 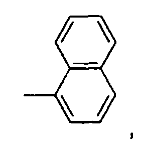 in particular a group of formula 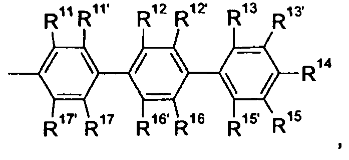 such as 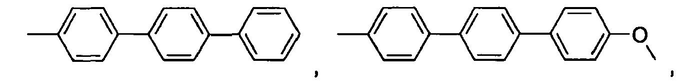 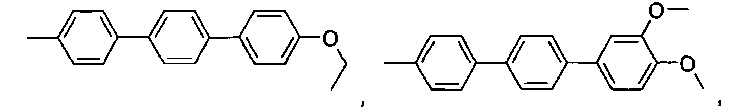 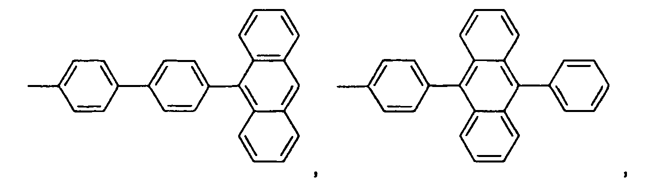 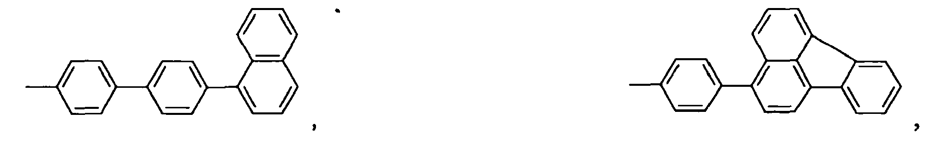 or 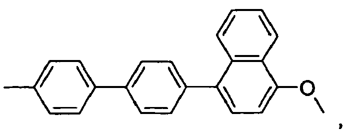
wherein R¹¹, R^{11'}, R¹², R^{12'}, R¹³, R^{13'}, R¹⁴, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, R^{47'}, R⁴³ and R⁴⁸ are defined as in claim 1 and are especially H, C₁-C₈alkyl, C₁-C₈alkoxy, or phenyl, or
R¹³ and R^{13'}, R^{13'} and R¹⁴, R¹⁴ and R¹⁵, or R¹⁵ and R^{15'} can be a divalent group 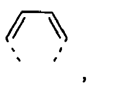 and Y¹ and Y² are a hydrogen atom; or
Y¹ and Y² are hydrogen and X¹ and X² are independently of each other a group Ar¹-Ar², wherein
Ar¹ is a group of formula 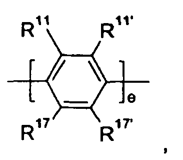
Ar² is a group of formula 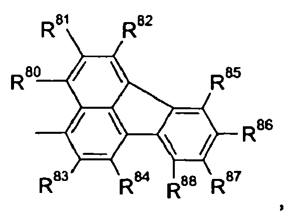 wherein
R⁸⁰, R⁸¹, R⁸², R⁸³, R⁸⁴, R⁸⁵, R⁸⁶, R⁸⁷ and R⁸⁸ are independently of each other H, E', C₆-C₁₈aryl; C₆-C₁₈aryl, which is substituted by E; C₁-C₁₈alkyl; C₁-C₁₈alkyl which is substituted by E' and/or interrupted by D; C₇-C₁₈aralkyl; or C₇-C₁₈aralkyl which is substituted by E;
e is an integer 1, or 2, and R¹¹, R^{11'}, R¹⁷ and R^{17'} are as defined in claim 1; or
Y¹ and Y² are independently of each other a group of the formula -W¹ -(W²)_{b}-W³, wherein b is 0, or, 1, especially hydrogen, and X¹ and X² are independently of each other a group -W¹ -(W²)_{b}-W³, wherein
W¹ and W² are independently of each other a group of formula 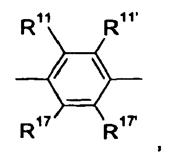 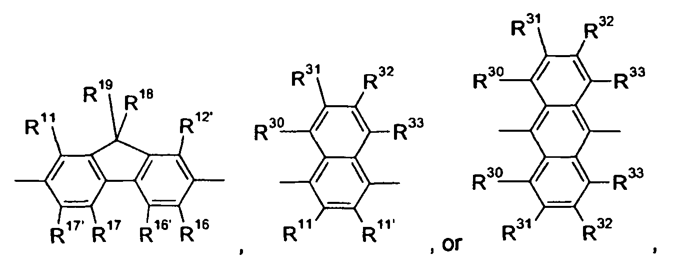
W³ is a group of formula 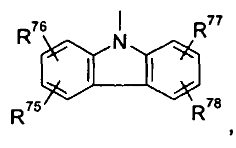 or -NR⁷⁰R⁷¹, wherein R⁷⁰ and R⁷¹ are independently of each other a group of formula 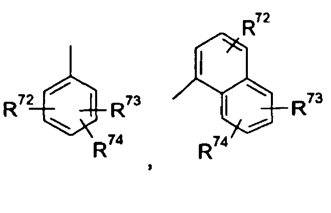 or 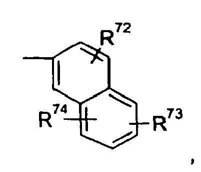 wherein R⁷², R⁷³ and R⁷⁴ are independently of each other hydrogen, C₁-C₈alkyl, C₁-C₈alkoxy, C₁-C₈alkylthio, a cyano group, a carbamoyl group, an amino group, a silyl group or a siloxanyl group,
R⁷⁵, R⁷⁶, R⁷⁷ and R⁷⁸ are independently of each other H, E, C₆-C₁₈aryl; C₆-C₁₈aryl which is substituted by C₁-C₁₈alkoxy, C₁-C₁₈alkyl, C₁-C₁₈alkyl which is interrupted by -O-; C₇-C₁₈aralkyl; or C₇-C₁₈aralkyl which is substituted by C₁-C₁₈alkoxy; wherein D, E, E', R¹¹, R^{11'}, R^{12'}, R¹⁶, R^{16'}, R¹⁷, R^{17'}, R¹⁸, R¹⁹, R³⁰, R³¹ , R³² and R³³ are as defined in claim 1.

7. An electroluminescent device, comprising a pyrazine compound of formula I according to any of claims 1 to 6.

8. The electroluminescent device according to claim 7, wherein the electroluminescent device comprises in this order
(a) an anode
(b) a hole injecting layer and/or a hole transporting layer
(c) a light-emitting layer
(d) optionally an electron transporting layer and
(e) a cathode.

9. The electroluminescent device according to claim 8, wherein the pyrazine compound of formula I forms the light-emitting layer.

10. Use of the pyrazine compounds of formula I according to any of claims 1 to 6 for electrophotographic photoreceptors, photoelectric converters, solar cells, image sensors, dye lasers and electroluminescent devices.

## Patentansprüche

1. Pyrazinverbindung der Formel 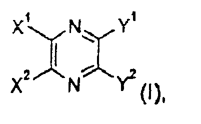 worin
X¹ eine Gruppe der Formel 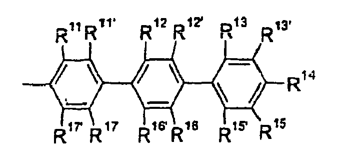 oder eine C₁₆-C₃₀-Arylgruppe ist, die gegebenenfalls durch E substituiert sein kann;
X² eine Arylgruppe oder eine Heteroarylgruppe ist, die gegebenenfalls substituiert sein kann; insbesondere eine Gruppe der Formel 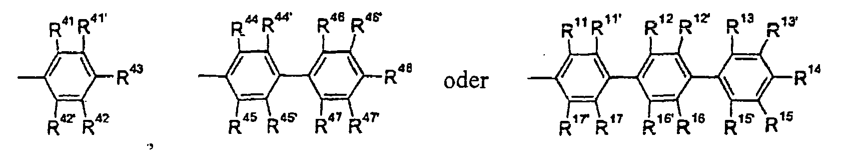 oder eine C₁₆-C₃₀-Arylgruppe, die gegebenenfalls durch E substituiert sein kann;
Y¹ und Y² unabhängig voneinander ein Wasserstoffatom, C₁-C₁₈-Alkyl, das gegebenenfalls durch O unterbrochen ist, eine Arylgruppe oder eine Heteroarylgruppe, die gegebenenfalls substituiert sein kann; insbesondere eine C₁₆-C₃₀-Arylgruppe, die gegebenenfalls durch E substituiert sein kann; oder eine Gruppe der Formel 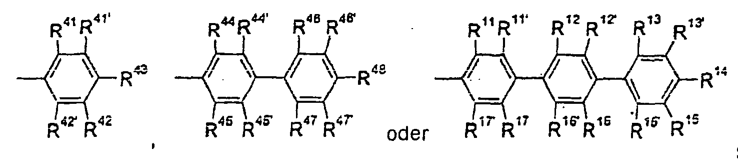 sind, oder
Y¹ und Y² zusammen eine C₅-C₈-Cycloalkylgruppe bilden, worin
R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ und R^{47'} unabhängig voneinander H, E, C₆-C₁₈-Aryl; C₆-C₁₈-Aryl, das durch E substituiert ist; C₁-C₁₈-Alkyl; C₁-C₁₈-Alkyl, das durch E' substituiert und/oder durch D unterbrochen ist; C₇-C₁₈-Aralkyl; oder C₇-C₁₈-Aralkyl, das durch E substituiert ist, sind; oder
R^{11'} und R¹², R^{12'} und R¹³, R^{15'} und R¹⁶, R^{16'} und R¹⁷, R^{44'} und R⁴⁶ und/oder R^{45'} und R⁴⁷ jeweils eine zweiwertige Gruppe L¹ sind, ausgewählt aus einem Sauerstoffatom, einem Schwefelatom, >CR¹⁸R¹⁹, >SiR¹⁸R¹⁹ oder 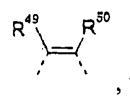 worin
R¹⁸ und R¹⁹ unabhängig voneinander C₁-C₁₈-Alkyl; C₁-C₁₈-Alkoxy, C₆-C₁₈-Aryl, C₆-C₁₈-Aryl, das durch E substituiert ist; C₇-C₁₈-Aralkyl oder C₇-C₁₈-Aralkyl, das durch E substituiert ist, sind; oder
R¹¹ und R^{11'}, R¹² und R^{12'}, R¹³ und R^{13'}, R^{13'} und R¹⁴, R¹⁴ und R¹⁵, R¹⁵ und R^{15'}, R¹⁶ und R^{16'}, R^{17'} und R¹⁷, R⁴¹ und R^{41'}, R⁴² und R^{42'}, R^{42'} und R⁴³, R^{41'} und R⁴³, R⁴⁴ und R^{44'}, R⁴⁵ und R^{45'}, R⁴⁶ und R^{46'}, R⁴⁷ und R^{47'}, R^{46'} und R⁴⁸ und/oder R^{47'} und R⁴⁸ jeweils eine zweiwertige Gruppe 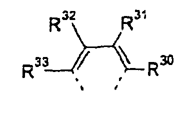 sind, worin
R³⁰, R³¹, R³², R³³, R⁴⁹ und R⁵⁰ unabhängig voneinander H, C₁-C₁₈-Alkyl; C₁-C₁₈-Alkyl, das durch E' substituiert und/oder durch D unterbrochen ist; E; C₆-C₁₈-Aryl; C₆-C₁₈-Aryl, das durch E substituiert ist, sind;
R¹⁴ H, C₂-C₃₀-Heteroaryl, -NR⁷⁰R⁷¹, C₆-C₃₀-Aryl oder C₆-C₃₀-Aryl, das durch E substituiert ist; C₁-C₁₈-Alkyl oder C₁-C₁₈-Alkyl, das durch E' substituiert und/oder durch D unterbrochen ist, ist; insbesondere 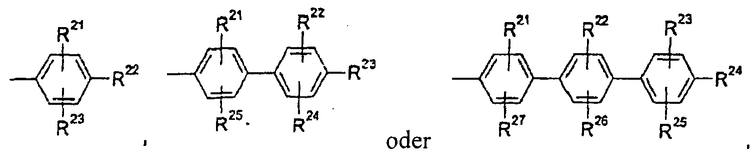 worin
R²¹, R²², R²³, R²⁴, R²⁵, R²⁶ und R²⁷ unabhängig voneinander H, E, C₁-C₁₈-Alkyl; C₁-C₁₈-Alkyl, das durch E' substituiert und/oder durch D unterbrochen ist; E; C₇-C₁₈-Aralkyl; C₇-C₁₈-Aralkyl, das durch E substituiert ist, sind;
R⁴³ und R⁴⁸ unabhängig voneinander H, E; insbesondere C₁-C₂₄-Alkyl, C₁-C₂₄-Alkoxy oder -NR⁷⁰R⁷¹ sind, worin R⁷⁰ und R⁷¹ unabhängig voneinander H, C₆-C₁₈-Aryl, C₆-C₁₈-Aryl, das durch C₁-C₂₄-Alkyl oder C₁-C₂₄-Alkoxy substituiert ist; C₁-C₂₄-Alkyl oder C₁-C₂₄-Alkyl, das durch -O- unterbrochen ist, sind, oder
R⁷⁰ und R⁷¹ zusammen einen fünf- oder sechsgliedrigen Ring bilden, insbesondere 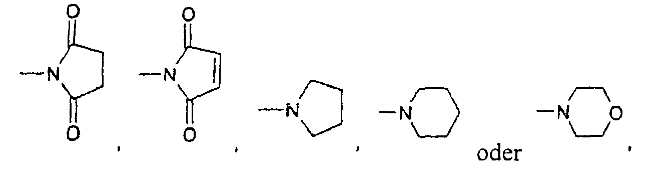 C₁-C₁₈-Alkyl; C₁-C₁₈-Alkyl, das durch E substituiert und/oder durch D unterbrochen ist; C₂-C₃₀-Heteroaryl; C₇-C₁₈-Aralkyl; C₇-C₁₈-Aralkyl, das durch E substituiert ist;
D -CO-; -COO-; -OCOO-; -S-; -SO-; -SO₂-; -O-; -NR⁵-; -SiR⁶¹R⁶²-; -POR⁵-; -CR⁶³=CR⁶⁴-oder -C≡C- ist;
E C₁-C₁₈-Alkyl, -OR⁵, -SR⁵; -NR⁵R⁶; -COR⁸; -COOR⁷; -CONR⁵R⁶; -CN oder Halogen ist; E' E ist, außer C₁-C₁₈-Alkyl, worin
R⁵ und R⁶ unabhängig voneinander C₆-C₁₈-Aryl; C₆-C₁₈-Aryl, das durch C₁-C₁₈-Alkyl oder C₁-C₁₈-Alkoxy substituiert ist; C₁-C₁₈-Alkyl, oder C₁-C₁₈-Alkyl, das durch -O- unterbrochen ist, sind; oder
R⁵ und R⁶ zusammen einen fünf- oder sechsgliedrigen Ring bilden, insbesondere 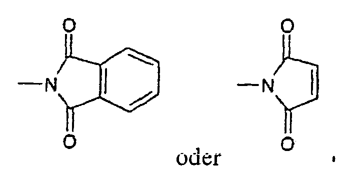
R⁷ C₆-C₁₈-Aryl; C₆-C₁₈-Aryl, das durch C₁-C₁₈-Alkyl oder C₁-C₁₈-Alkoxy substituiert ist; C₁-C₁₈-Alkyl oder C₁-C₁₈-Alkyl, das durch -O- unterbrochen ist, ist;
R⁸ C₇-C₁₂-Alkylaryl; C₁-C₁₈-Alkyl oder C₁-C₁₈-Alkyl, das durch -O- unterbrochen ist, ist; R⁶¹ und R⁶² unabhängig voneinander C₆-C₁₈-Aryl; C₆-C₁₈-Aryl, das durch C₁-C₁₈-Alkyl oder C₁-C₁₈-Alkoxy substituiert ist; C₁-C₁₈-Alkyl oder C₁-C₁₈-Alkyl, das durch -O- unterbrochen ist, sind; und
R⁶³ und R⁶⁴ unabhängig voneinander H, C₆-C₁₈-Aryl; C₆-C₁₈-Aryl, das durch C₁-C₁₈-Alkyl oder C₁-C₁₈-Alkoxy substituiert ist; C₁-C₁₈-Alkyl oder C₁-C₁₈-Alkyl, das durch -O- unterbrochen ist, sind.

2. Pyrazinverbindung der Formel I nach Anspruch 1, worin X¹ und X² unabhängig voneinander eine Gruppe der Formel 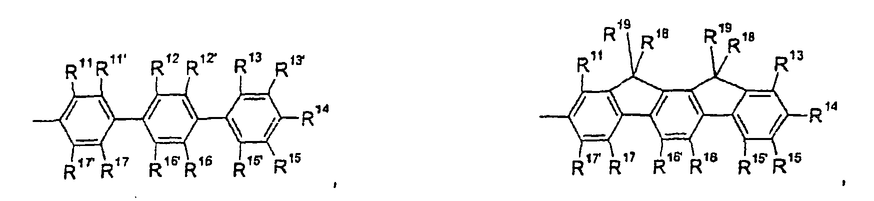 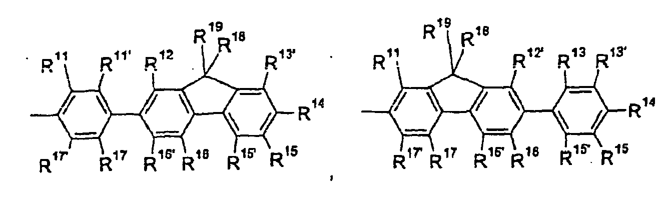 oder -X¹¹-X¹²-X¹³ sind, worin
R¹¹, R^{11'}, R¹², R^{12'}, R¹³, R^{13'}, R¹⁵, R^{15'}, R¹⁶, R^{16'}, R¹⁷ und R^{17'} unabhängig voneinander H, C₆-C₁₈-Aryl; C₆-C₁₈-Aryl, das durch E substituiert ist; E, C₁-C₁₈-Alkyl; C₁-C₁₈-Alkyl, das durch E' substituiert und/oder durch D unterbrochen ist; C₇-C₁₈-Aralkyl; C₇-C₁₈-Aralkyl, das durch E substituiert ist, sind; und
R¹⁴, R¹⁸ und R¹⁹ wie in Anspruch 1 definiert sind,
X¹¹ und X¹² unabhängig voneinander eine Gruppe der Formel 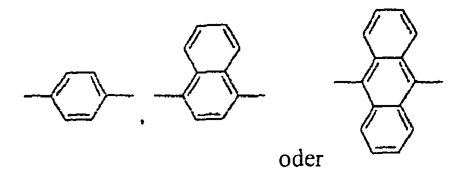
sind und X¹³ eine Gruppe der Formel 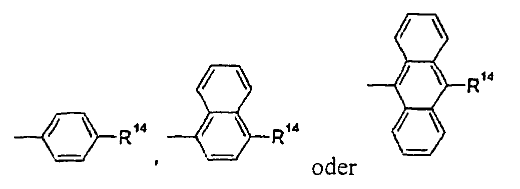 ist,
worin R¹⁴ 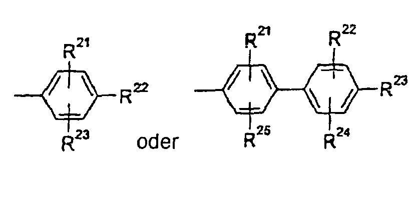 ist,
worin R²¹, R²², R²³, R²⁴ und R²⁵ wie in Anspruch 1 definiert sind, und Y¹ und Y² ein Wasserstoffatom, C₁-C₁₈-Alkyl, das gegebenenfalls durch O unterbrochen ist, sind, oder Y¹ und Y² zusammen eine C₅-C₈-Cycloalkylgruppe bilden und insbesondere ein Wasserstoffatom sind.

3. Pyrazinverbindung nach Anspruch 1 oder 2, worin R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ und R^{47'} sowie R¹⁴, R⁴³ und R⁴⁸ unabhängig voneinander H, E oder C₁-C₈-Alkyl sind; worin E -OR⁵; -SR⁵; -NR⁵R⁶; -COR⁸; -COOR⁷; -CONR⁵R⁶; -CN; -OCOOR⁷ oder Halogen ist; worin R⁵ und R⁶ unabhängig voneinander C₆-C₁₂-Aryl oder C₁-C₈-Alkyl sind;
R⁷ C₇-C₁₂-Alkylaryl oder C₁-C₈-Alkyl ist; und
R⁸ C₆-C₁₂-Aryl oder C₁-C₈-Alkyl ist; oder
R¹¹ und R^{11'}, R¹² und R^{12'}, R¹³ und R^{13'}, R^{13'} und R¹⁴, R⁴¹ und R^{41'}, R^{41'} und R⁴³, R⁴⁴ und R^{44'}, R⁴⁶ und R^{46'}, R^{46'} und R⁴⁸ und/oder R^{47'} und R⁴⁸ jeweils eine zweiwertige Gruppe 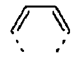 sind.

4. Pyrazinverbindung nach Anspruch 1, worin
X¹ und X² eine Gruppe der Formel 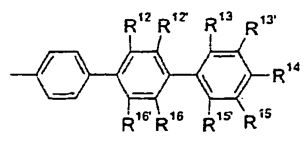 sind, worin
R¹³, R^{13'}, R¹⁵ und R^{15'} H sind, und R¹⁴ H oder 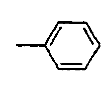 ist, und R¹² R^{12'}, R¹⁶ und R^{16'} H sind; oder
R¹³ und R¹⁵ H sind, R^{13'} und R^{15'} unabhängig voneinander H, C₁-C₈-Alkyl oder C₁-C₈-Alkoxy sind, und R¹⁴ H, C₁-C₈-Alkyl oder C₁-C₈-Alkoxy ist, und R¹², R^{12'}, R¹⁶ und R^{16'} H sind, worin zumindest einer von R¹³, R¹⁵, R^{13'}, R^{15'} und R¹⁴ C₁-C₈-Alkyl oder C₁-C₈-Alkoxy ist;
R¹² und R^{12'}, R¹³ und R^{13'}, R^{13'} und R¹⁴, R¹⁴ und R¹⁵, R¹⁵ und R^{15'}, und/oder R¹⁶ und R^{16'} eine zweiwertige Gruppe 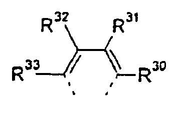 sein können; oder
R^{12'}, R¹⁶, R^{16'} H sind und R¹³ und R^{13'} oder R^{13'} und R¹⁴ und/oder R¹⁵ und R^{15'} eine zweiwertige Gruppe 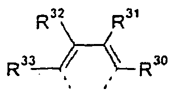 sind; oder
R¹³, R^{13'}, R¹⁴, R¹⁵, R^{15'} H sind, R¹⁴ H, C₁-C₈-Alkyl ist, und R¹² und R^{12'} und/oder R¹⁶ und R^{16'} eine zweiwertige Gruppe 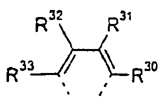 sind, worin
R³⁰, R³¹, R³² und R³³ H, C₁-C₈-Alkyl oder C₁-C₈-Alkoxy sind, und
Y¹ und Y² ein Wasserstoffatom sind.

5. Pyrazinverbindung nach einem der Ansprüche 1 bis 3, worin
X¹ und X² unabhängig voneinander eine Gruppe der Formel 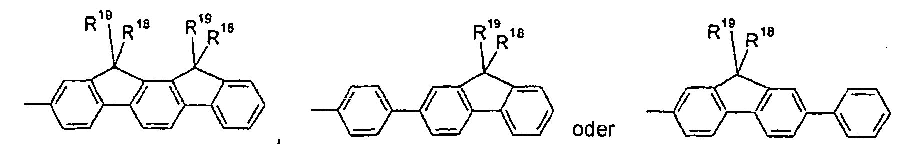 sind, worin R¹⁸ und R¹⁹ unabhängig voneinander C₁-C₈-Alkyl sind.

6. Pyrazinverbindung nach Anspruch 1, worin
X¹ ist eine Gruppe der Formel 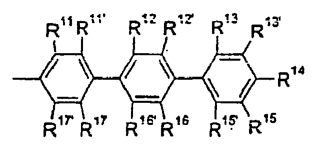 ist, insbesondere 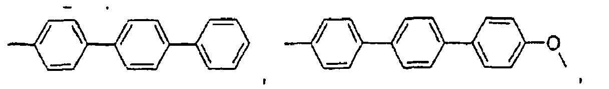 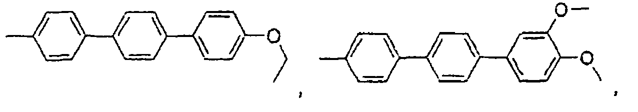 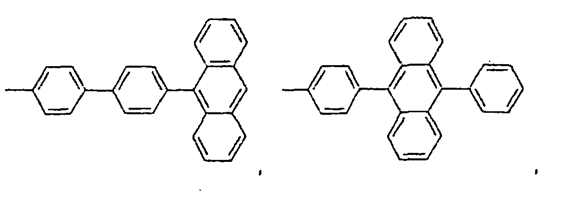 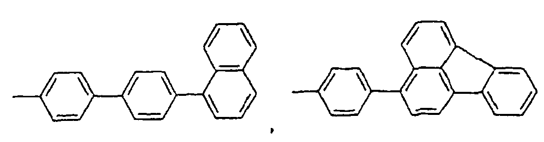 oder 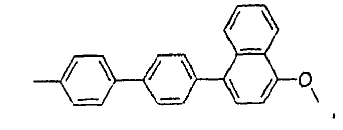 und
X² eine Gruppe der Formel 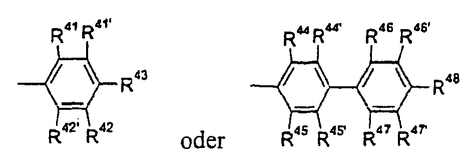 ist, insbesondere 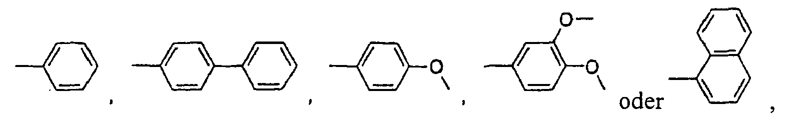 insbesondere eine Gruppe der Formel 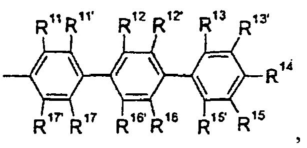 wie 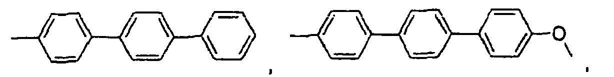 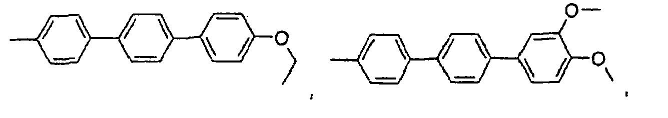 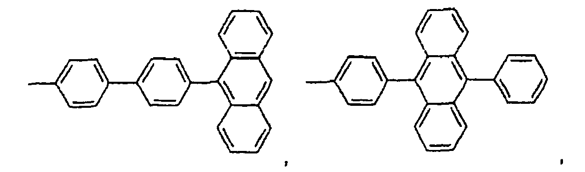 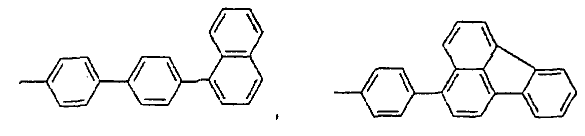 oder 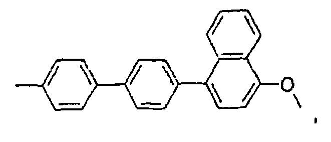 worin R¹¹, R^{11'}, R¹², R^{12'}, R¹³, R^{13'}, R¹⁴, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, R^{47'}, R⁴³ und R⁴⁸ wie in Anspruch 1 definiert sind und insbesondere H, C₁-C₈-Alkyl, C₁-C₈-Alkoxy oder Phenyl sind, oder
R¹³ und R^{13'}, R^{13'} und R¹⁴, R¹⁴ und R¹⁵, oder R¹⁵ und R^{15'} eine zweiwertige Gruppe 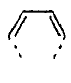 sein können,
und Y¹ und Y² ein Wasserstoffatom sind; oder
Y¹ und Y² Wasserstoff sind und X¹ und X² unabhängig voneinander eine Gruppe Ar¹-Ar² sind, worin
Ar¹ eine Gruppe der Formel 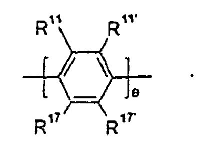 ist,
Ar² eine Gruppe der Formel 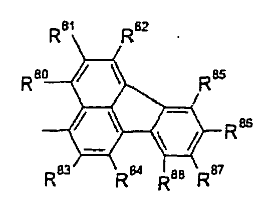 ist, worin
R⁸⁰, R⁸¹,R⁸², R⁸³, R⁸⁴, R⁸⁵, R⁸⁶, R⁸⁷ und R⁸⁸ unabhängig voneinander H, E', C₆-C₁₈-Aryl; C₆-C₁₈-Aryl, das durch E substituiert ist; C₁-C₁₈-Alkyl; C₁-C₁₈-Alkyl, das durch E' substituiert und/oder durch D unterbrochen ist; C₇-C₁₈-Aralkyl; oder C₇-C₁₈-Aralkyl, das durch E substituiert ist, sind;
e eine ganze Zahl 1 oder 2 ist, und R¹¹, R^{11'}, R¹⁷ und R^{17'} wie in Anspruch 1 definiert sind; oder
Y¹ und Y² unabhängig voneinander eine Gruppe der Formel -W¹-(W²)_{b}-W³ sind, worin b 0 oder 1 ist, insbesondere Wasserstoff, und X¹ und X² unabhängig voneinander eine Gruppe -W¹-(W²)_{b}-W³ sind, worin
W¹ und W² sind unabhängig voneinander eine Gruppe der Formel 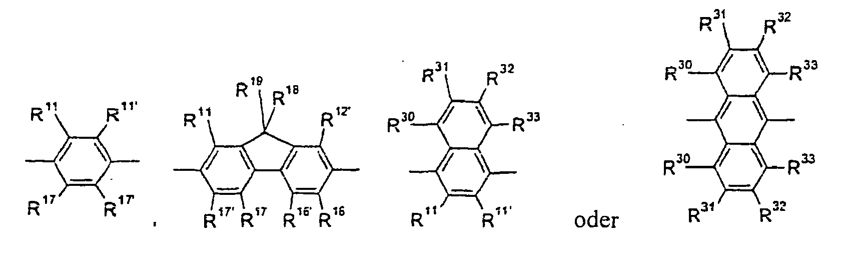 sind,
W³ eine Gruppe der Formel 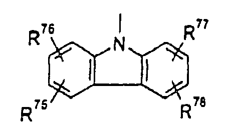 oder -NR⁷⁰R⁷¹ ist,
worin R⁷⁰ und R⁷¹ unabhängig voneinander eine Gruppe der Formel 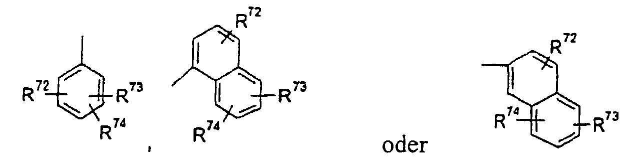 sind,
worin R⁷², R⁷³ und R⁷⁴ unabhängig voneinander Wasserstoff, C₁-C₈-Alkyl, C₁-C₈-Alkoxy, C₁-C₈-Alkylthio, eine Cyanogruppe, eine Carbamoylgrupe, eine Aminogruppe, eine Silylgruppe oder eine Siloxanylgruppe sind,
R⁷⁵, R⁷⁶, R⁷⁷ und R⁷⁸ unabhängig voneinander H, E, C₆-C₁₈-Aryl; C₆-C₁₈-Aryl, das durch C₁-C₁₈-Alkoxy substituiert ist, C₁-C₁₈-Alkyl, C₁-C₁₈-Alkyl, das durch -O- unterbrochen ist; C₇-C₁₈-Aralkyl; oder C₇-C₁₈-Aralkyl, das durch C₁-C₁₈-Alkoxy substituiert ist, sind; worin D, E, E', R¹¹,R^{11'},R^{12'}, R¹⁶, R^{16'}, R¹⁷, R^{17'}, R¹⁸, R¹⁹, R³⁰, R³¹, R³² und R³³ wie in Anspruch 1 definiert sind.

7. Elektrolumineszenzvorrichtung, umfassend eine Pyrazinverbindung der Formel I nach einem der Ansprüche 1 bis 6.

8. Elektrolumineszenzvorrichtung nach Anspruch 7, wobei die Elektrolumineszenzvorrichtung in dieser Reihenfolge
(a) eine Anode,
(b) eine Lochinjektionsschicht und/oder eine Lochtransportschicht,
(c) eine lichtemittierende Schicht,
(d) gegebenenfalls eine Elektronentransportschicht und
(e) eine Kathode
umfaßt.

9. Elektrolumineszenzvorrichtung nach Anspruch 8, wobei die Pyrazinverbindung der Formel I die lichtemittierende Schicht bildet.

10. Verwendung der Pyrazinverbindungen der Formel I nach einem der Ansprüche 1 bis 6 für elektrophotografische Photorezeptoren, photoelektrische Konverter, Solarzellen, Bildsensoren, Farbstofflaser und Elektrolumineszenzvorrichtungen.

## Revendications

1. Composé pyrazine de formule : 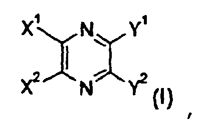 dans laquelle
X¹ représente un groupe de formule 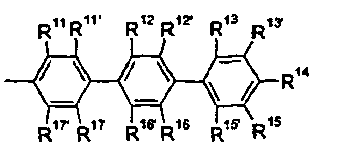 ou un groupe aryle en C₁₆ à C₃₀, qui peut éventuellement être substitué par E ;
X² représente un groupe aryle ou un groupe hétéroaryle qui peut éventuellement être substitué ; en particulier un groupe de formule 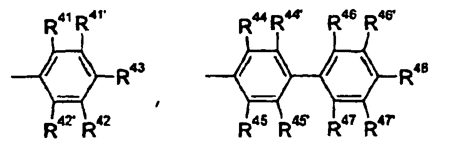 ou 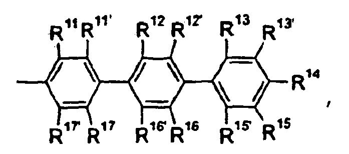 ou un groupe aryle en C₁₆ à C₃₀, qui peut éventuellement être substitué par E ;
Y¹ et Y² représentent chacun, indépendamment l'un de l'autre, un atome d'hydrogène, un groupe alkyle en C₁ à C₁₈ qui est éventuellement interrompu par O,
un groupe aryle ou un groupe hétéroaryle qui peut éventuellement être substitué ; en particulier un groupe aryle en C₁₆ à C₃₀ qui peut éventuellement être substitué par E ; ou un groupe de formule 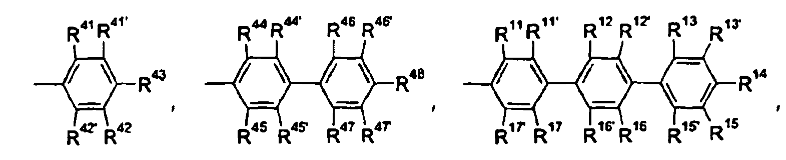 ou
Y¹ et Y² forment ensemble un groupe cycloalkyle en C₅ à C₈, dans lequel
R¹¹, R^{11'}, R¹² R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ et R^{47'} représentent chacun, indépendamment les uns des autres, un atome d'hydrogène, E, un groupe aryle en C₆ à C₁₈ ; aryle en C₆ à C₁₈ qui est substitué par E ; alkyle en C₁ à C₁₈ ; alkyle en C₁ à C₁₈ qui est substitué par E' et/ou interrompu par D ; aralkyle en C₇ à C₁₈ ; ou aralkyle en C₇ à C₁₈ qui est substitué par E ; ou
R^{11'} et R¹², R^{12'} et R¹³, R^{15'} et R¹⁶, R^{16'} et R¹⁷, R^{44'} et R⁴⁶ et/ou R^{45'} et R⁴⁷ représentent chacun un groupe bivalent L¹ choisi parmi un atome d'oxygène, un atome de soufre, >CR¹⁸R¹⁹ >SiR¹⁸R¹⁹, ou 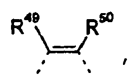 où
R¹⁸ et R¹⁹ représentent chacun, indépendamment l'un de l'autre, un groupe alkyle en C₁ à C₁₈ ; alcoxyle en C₁ à C₁₈, aryle en C₆ à C₁₈, aryle en C₆ à C₁₈ qui est substitué par E ; aralkyle en C₇ à C₁₈, ou aralkyle en C₇ à C₁₈ qui est substitué par E ; ou
R¹¹ et R^{11'}, R¹² et R^{12'}, R¹³ et R^{13'}, R^{13'} et R¹⁴, R¹⁴ et R¹⁵, R¹⁵ et R^{15'}, R¹⁶ et R^{16'}, R¹⁷ et R^{17'}, R⁴¹ et R^{41'}, R⁴² et R^{42'}, R^{42'} et R⁴³, R^{41'} et R⁴³, R⁴⁴ et R^{44'}, R⁴⁵ et R^{45'}, R⁴⁶ et R^{46'}, R⁴⁷ et R^{47'}, R^{46'} et R⁴⁸ et/ou R^{47'} et R⁴⁸ représentent chacun un groupe bivalent 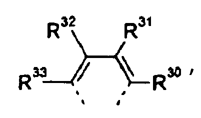 dans lequel
R³⁰, R³¹, R³², R³³, R⁴⁹ et R⁵⁰ représentent chacun, indépendamment les uns des autres, un atome d'hydrogène, un groupe alkyle en C₁ à C₁₈ ; alkyle en C₁ à C₁₈ qui est substitué par E' et/ou interrompu par D ; E ; un groupe aryle en C₆ à C₁₈ ; aryle en C₆ à C₁₈ qui est substitué par E ;
R¹⁴ représente un atome d'hydrogène, un groupe hétéroaryle en C₂ à C₃₀, -NR⁷⁰R⁷¹, un groupe aryle en C₆ à C₃₀, ou aryle en C₆ à C₃₀ qui est substitué par E, alkyle en C₁ à C₁₈ ; ou alkyle en C₁ à C₁₈ qui est substitué par E' et/ou interrompu par D ; en particulier 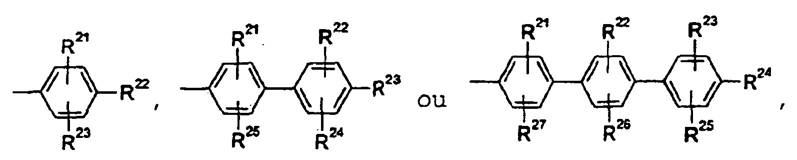 où
R²¹, R²², R²³, R²⁴, R²⁵, R²⁶ et R²⁷ représentent chacun, indépendamment les uns des autres, un atome d'hydrogène, E, un groupe alkyle en C₁ à C₁₈ ; alkyle en C₁ à C₁₈ qui est substitué par E' et/ou interrompu par D ; E ; aralkyle en C₇ à C₁₈ ; aralkyle en C₇ à C₁₈ qui est substitué par E ;
R⁴³ et R⁴⁸ représentent chacun, indépendamment l'un de l'autre, un atome d'hydrogène, E ; en particulier un groupe alkyle en C₁ à C₂₄, alcoxyle en C₁ à C₂₄ ou -NR⁷⁰R⁷¹, où R⁷⁰ et R⁷¹ représentent chacun, indépendamment l'un de l'autre, un atome d'hydrogène, un groupe aryle en C₆ à C₁₈, aryle en C₆ à C₁₈ qui est substitué par un groupe alkyle en C₁ à C₂₄ ou alcoxyle en C₁ à C₂₄ ; un groupe alkyle en C₁ à C₂₄, ou alkyle en C₁ à C₂₄ qui est interrompu par -O-, ou
R⁷⁰ et R⁷¹ forment ensemble un cycle à cinq ou six éléments, en particulier 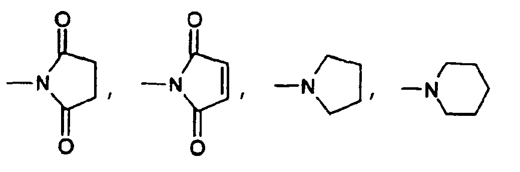 ou 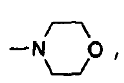 un groupe alkyle en C₁ à C₁₈ ; alkyle en C₁ à C₁₈ qui est substitué par E et/ou interrompu par D ; hétéroaryle en C₂ à C₃₀ ; aralkyle en C₇ à C₁₈ ; aralkyle en C₇ à C₁₈ qui est substitué par E ;
D représente -CO- ; -COO- ; -OCOO- ; -S- ; -SO- ; -SO₂- ; -O- ; -NR⁵- ; -SiR⁶¹R⁶²- ; -POR⁵- ; -CR⁶³=CR⁶⁴- ; ou -C≡C- ;
E représente un groupe alkyle en C₁ à C₁₈, -OR⁵ ; -SR⁵ ; -NR⁵R⁶ ; -COR⁸ ; -COOR⁷ ; -CONR⁵R⁶ ; -CN ; ou un atome d'halogène ;
E' représente E, sauf un groupe alkyle en C₁ à C₁₈, dans lequel
R⁵ et R⁶ représentent chacun, indépendamment l'un de l'autre, un groupe aryle en C₆ à C₁₈ ; aryle en C₆ à C₁₈ qui est substitué par un groupe alkyle en C₁ à C₁₈, ou alcoxyle en C₁ à C₁₈ ; alkyle en C₁ à C₁₈, ou alkyle en C₁ à C₁₈ qui est interrompu par -O- ; ou
R⁵ et R⁶ forment ensemble un cycle à cinq ou six éléments, en particulier 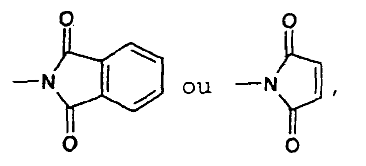
R⁷ représente un groupe aryle en C₆ à C₁₈ ; aryle en C₆ à C₁₈ qui est substitué par un groupe alkyle en C₁ à C₁₈, ou alcoxyle en C₁ à C₁₈ ; alkyle en C₁ à C₁₈ ; ou alkyle en C₁ à C₁₈ qui est interrompu par -O- ;
R⁸ représente un groupe alkylaryle en C₇ à C₁₂ ; alkyle en C₁ à C₁₈ ; ou alkyle en C₁ à C₁₈ qui est interrompu par -O- ;
R⁶¹ et R⁶² représentent chacun, indépendamment l'un de l'autre, un groupe aryle en C₆ à C₁₈ ; aryle en C₆ à C₁₈ qui est substitué par un groupe alkyle en C₁ à C₁₈, ou alcoxyle en C₁ à C₁₈ ; alkyle en C₁ à C₁₈, ou alkyle en C₁ à C₁₈ qui est interrompu par -O- ; et
R⁶³ et R⁶⁴ représentent chacun, indépendamment l'un de l'autre, un atome d'hydrogène, un groupe aryle en C₆ à C₁₈ ; aryle en C₆ à C₁₈ qui est substitué par un groupe alkyle en C₁ à C₁₈, ou alcoxyle en C₁ à C₁₈ ; alkyle en C₁ à C₁₈ ou alkyle en C₁ à C₁₈ qui est interrompu par -O-.

2. Composé pyrazine de formule I selon la revendication 1, dans lequel X¹ et X² représentent chacun, indépendamment l'un de l'autre, un groupe de formule : 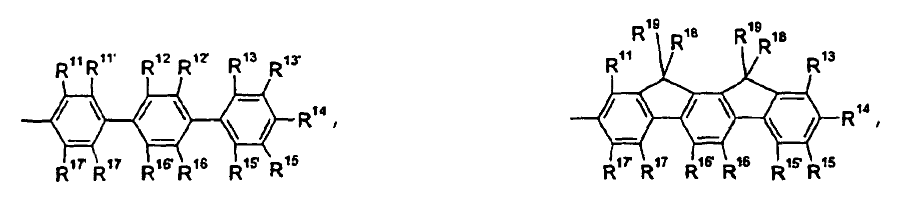 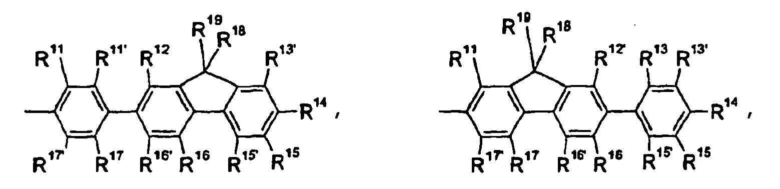 ou
-X¹¹-X¹²-X¹³, où
R¹¹, R^{11'}, R¹², R^{12'}, R¹³, R^{13'}, R¹⁵, R^{15'}, R¹⁶, R^{16'}, R¹⁷ et R^{17'} représentent chacun, indépendamment les uns des autres, un atome d'hydrogène, un groupe aryle en C₆ à C₁₈ ; aryle en C₆ à C₁₈ qui est substitué par E ; E, un groupe alkyle en C₁ à C₁₈ ; alkyle en C₁ à C₁₈ qui est substitué par E' et/ou interrompu par D ; aralkyle en C₇ à C₁₈ ; aralkyle en C₇ à C₁₈ qui est substitué par E ; et R¹⁴, R¹⁸ et R¹⁹ sont tels que définis dans la revendication 1,
X¹¹ et X¹² représentent chacun, indépendamment l'un de l'autre, un groupe de formule 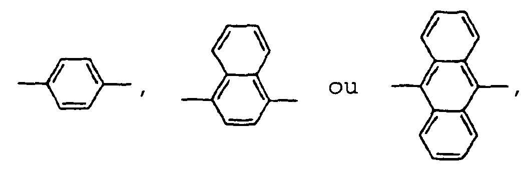 et X¹³ représente un groupe de formule 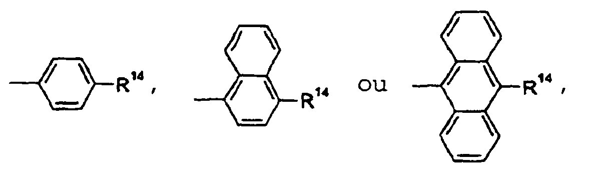 dans laquelle R¹⁴ représente 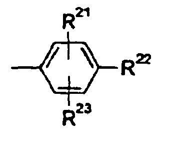 ou 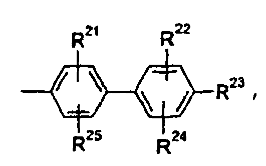 où R²¹, R²², R²³, R²⁴ et R²⁵ sont tels que définis dans la revendication 1 et Y¹ et Y² représentent chacun un atome d'hydrogène, un groupe alkyle en C₁ à C₁₈, qui est éventuellement interrompu par O, ou Y¹ et Y² forment ensemble un groupe cycloalkyle en C₅ à C₈, et ils représentent en particulier un atome d'hydrogène.

3. Composé pyrazine selon la revendication 1 ou 2, dans lequel R¹¹, R^{11'}, R¹², R^{12'}, R¹³, R^{13'}, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷ et R^{47'} ainsi que R¹⁴, R⁴³ et R⁴⁸ représentent chacun, indépendamment les uns des autres, un atome d'hydrogène, E ; ou un groupe alkyle en C₁ à C₈ ; où E représente -OR⁵ ; -SR⁵ ; -NR⁵R⁶ ; -COR⁸ ; -COOR⁷ ; -CONR⁵R⁶ ; -CN ; -OCOOR⁷ ; ou un atome d'halogène ; où R⁵ et R⁶ représentent chacun, indépendamment l'un de l'autre, un groupe aryle en C₆ à C₁₂ ou alkyle en C₁ à C₈ ;
R⁷ représente un groupe alkylaryle en C₇ à C₁₂ ou alkyle en C₁ à C₈ ; et
R⁸ représente un groupe aryle en C₆ à C₁₂ ; ou un groupe alkyle en C₁ à C₈ ; ou
R¹¹ et R^{11'}, R¹² et R^{12'}, R¹³ et R^{13'}, R^{13'} et R¹⁴, R⁴¹ et R^{41'}, R^{41'} et R^{43,} R⁴⁴ et R^{44'}, R⁴⁶ et R^{46'}, R^{46'} et R⁴⁸ et/or R^{47'} et R⁴⁸ représentent chacun un groupe bivalent

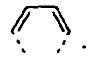

4. Composé pyrazine selon la revendication 1, dans lequel
X¹ et X² représentent un groupe de formule 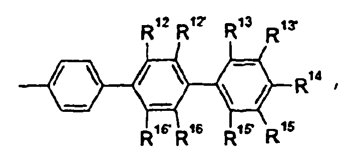 dans laquelle
R¹³, R^{13'}, R¹⁵ et R^{15'} représentent un atome d'hydrogène et R¹⁴ représente un atome d'hydrogène, ou 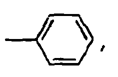 et
R¹², R^{12'}, R¹⁶ et R^{16'} représentent un atome d'hydrogène ; ou
R¹³ et R¹⁵ représentent un atome d'hydrogène, R^{13'} et R^{15'} représentent chacun, indépendamment l'un de l'autre, un atome d'hydrogène, un groupe alkyle en C₁ à C₈ ou alcoxyle en C₁ à C₈, et R¹⁴ représente un atome d'hydrogène, un groupe alkyle en C₁ à C₈ ou un groupe alcoxyle en C₁ à C₈, et R¹², R^{12'}, R¹⁶ et R^{16'} représentent un atome d'hydrogène, où au moins un parmi R¹³, R¹⁵, R^{13'}, R^{15'} et R¹⁴ représente un groupe alkyle en C₁ à C₈ ou alcoxyle en C₁ à C₈ ;
R¹² et R^{12'}, R¹³ et R^{13'}, R^{13'} et R¹⁴, R¹⁴ et R¹⁵, R¹⁵ et R^{15'}, et/ou R¹⁶ et R^{16'}, représentent chacun un groupe bivalent 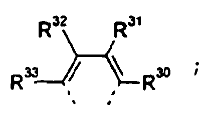 ou
R^{12'}, R¹⁶, R^{16'} représentent chacun un atome d'hydrogène et R¹³ et R^{13'}, ou R^{13'} et R¹⁴ et/ou R¹⁵ et R^{15'} représentent chacun un groupe bivalent 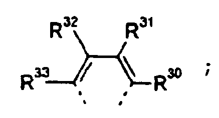 ou R¹³, R^{13'}, R¹⁴, R¹⁵, R^{15'} représentent un atome d'hydrogène, R¹⁴ représente un atome d'hydrogène, un groupe alkyle en C₁ à C₈ et R¹² et R^{12'}, et/ou R¹⁶ et
R^{16'} représentent chacun un groupe bivalent 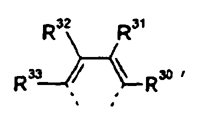 dans lequel R³⁰, R³¹, R³² et R³³ représentent un atome d'hydrogène, un groupe alkyle en C₁ à C₈ ou alcoxy en C₁ à C₈, et
Y¹ et Y² représentent chacun un atome d'hydrogène.

5. Composé pyrazine selon l'une quelconque des revendications 1 à 3, dans lequel
X¹ et X² représentent chacun, indépendamment l'un de l'autre, un groupe de formule 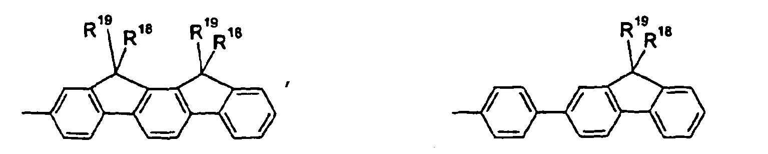 ou 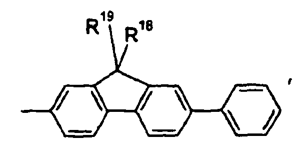 où
R¹⁸ et R¹⁹ représentent chacun, indépendamment l'un de l'autre, un groupe alkyle en C₁ à C₈.

6. Composé pyrazine selon la revendication 1, dans lequel
x¹ représente un groupe de formule 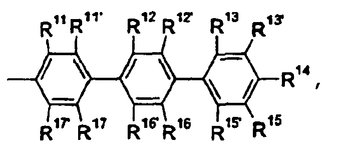 en particulier 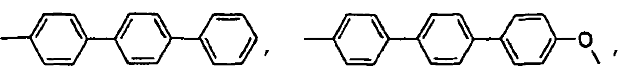 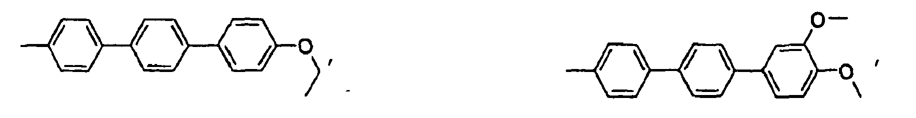 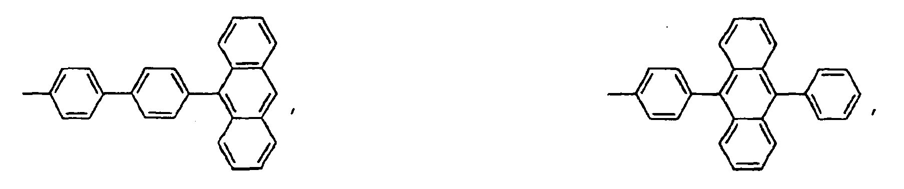 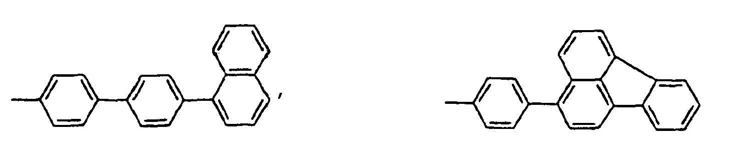 ou 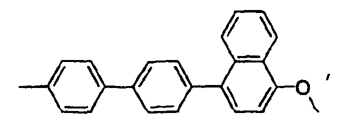 et
X² représente un groupe de formule 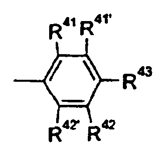 ou 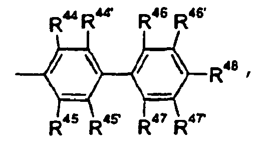 en particulier 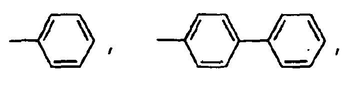 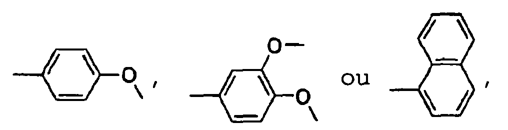 en particulier un groupe de formule 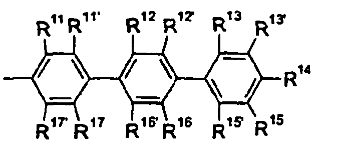 tel que 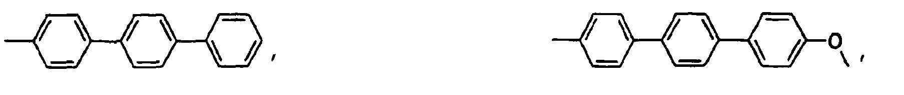 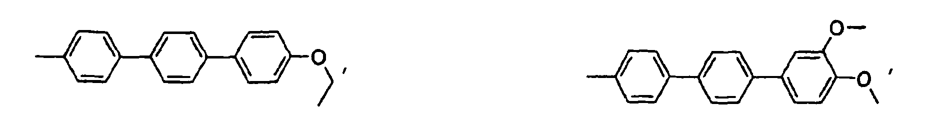 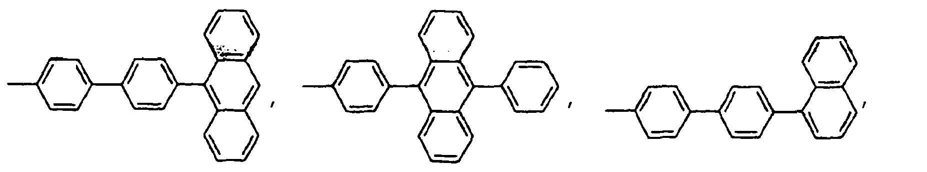 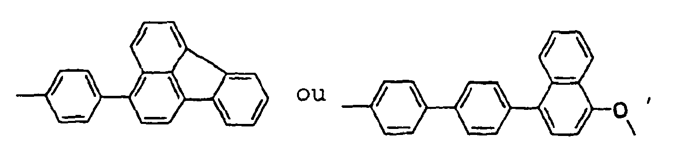
où R¹¹, R¹¹' R¹², R^{12'}, R¹³, R^{13'}, R¹⁴, R¹⁵, R^{15'}, R¹⁶, R^{16'}, R¹⁷, R^{17'}, R⁴¹, R^{41'}, R⁴², R^{42'}, R⁴⁴, R^{44'}, R⁴⁵, R^{45'}, R⁴⁶, R^{46'}, R⁴⁷, R^{47'}, R⁴³ et R⁴⁸ sont tels que définis dans la revendication 1 et ils représentent en particulier un atome d'hydrogène, un groupe alkyle en C₁ à C₈, alcoxyle en C₁ à C₈, ou phényle, ou
R¹³ et R^{13'}, R^{13'} et R¹⁴, R¹⁴ et R¹⁵, ou R¹⁵ et R^{15'} peuvent être un groupe bivalent 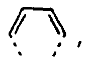 et Y¹ et Y² représentent chacun un atome d'hydrogène ; ou
Y¹ et Y² représentent chacun un atome d'hydrogène et X¹ et X² représentent chacun, indépendamment l'un de l'autre, un groupe Ar¹-Ar², dans lequel
Ar¹ représente un groupe de formule 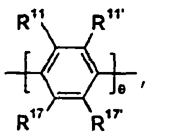
Ar² représente un groupe de formule 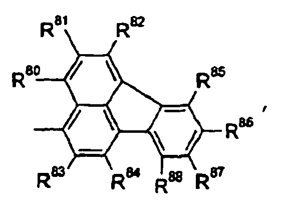 dans laquelle
R⁸⁰, R⁸¹, R⁸², R⁸³, R⁸⁴, R⁸⁵, R⁸⁶, R⁸⁷ et R⁸⁸ représentent chacun, indépendamment les uns des autres, un atome d'hydrogène, E', un groupe aryle en C₆ à C₁₈ ; aryle en C₆ à C₁₈ qui est substitué par E ; alkyle en C₁ à C₁₈ ; alkyle en C₁ à C₁₈ qui est substitué par E' et/ou interrompu par D ; aralkyle en C₇ à C₁₈ ; ou aralkyle en C₇ à C₁₈ qui est substitué par E ;
e représente le nombre entier 1 ou 2, et R¹¹, R^{11'}, R¹⁷ et R^{17'} sont tels que définis dans la revendication 1 ; ou
Y¹ et Y² représentent chacun, indépendamment l'un de l'autre, un groupe de formule W¹-(W²)_{b}-W³, dans laquelle b vaut 0 ou 1, en particulier un atome d'hydrogène, et X¹ et X² représentent chacun, indépendamment l'un de l'autre, un groupe -W¹-(W²)_{b}-W³, dans lequel
W¹ et W² représentent chacun, indépendamment l'un de l'autre, un groupe de formule 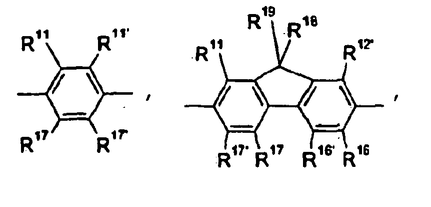 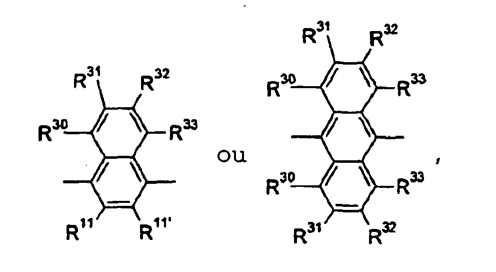
W³ représente un groupe de formule 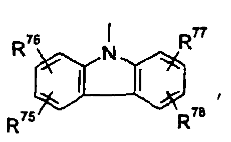 ou -NR⁷⁰R⁷¹, où R⁷⁰ et R⁷¹ représentent chacun, indépendamment l'un de l'autre, un groupe de formule 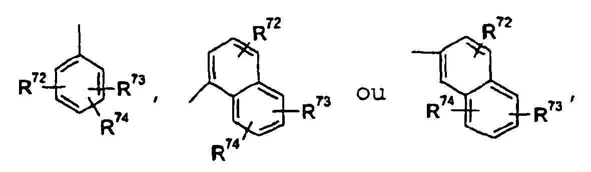 où R⁷², R⁷³ et R⁷⁴ représentent chacun, indépendamment les uns des autres, un atome d'hydrogène, un groupe alkyle en C₁ à C₈, alcoxyle en C₁ à C₈, alkylthio en C₁ à C₈, un groupe cyano, un groupe carbamoyle, un groupe amino, un groupe silyle ou un groupe siloxanyle,
R⁷⁵, R⁷⁶, R⁷⁷ et R⁷⁸ représentent chacun, indépendamment les uns des autres, un atome d'hydrogène, E, un groupe aryle en C₆ à C₁₈ ; aryle en C₆ à C₁₈ qui est substitué par un groupe alcoxyle en C₁ à C₁₈, alkyle en C₁ à C₁₈, alkyle en C₁ à C₁₈ qui est interrompu par -O- ; aralkyle en C₇ à C₁₈ ; ou aralkyle en C₇ à C₁₈ qui est substitué par un groupe alcoxyle en C₁ à C₁₈ ; où D, E, E', R¹¹, R^{11'}, R^{12'}, R¹⁶, R^{16'}, R¹⁷, R^{17'}, R¹⁸, R¹⁹, R³⁰, R³¹, R³² et R³³ sont tels que définis dans la revendication 1

7. Dispositif électroluminescent comprenant un composé pyrazine de formule I selon l'une quelconque des revendications 1 à 6.

8. Dispositif électroluminescent selon la revendication 7, dans lequel le dispositif électroluminescent comprend dans cet ordre :
(a) une anode
(b) une couche d'injection de trous et/ou une couche de transport de trous
(c) une couche émettrice de lumière
(d) éventuellement une couche de transport d'électrons et
(e) une cathode.

9. Dispositif électroluminescent selon la revendication 8, dans lequel le composé pyrazine de formule 1 forme la couche émettrice de lumière.

10. Utilisation des composés pyrazine de formule I selon l'une quelconque des revendications 1 à 6 pour photorécepteurs électrophotographiques, convertisseurs photoélectriques, cellules solaires, capteurs d'image, lasers à colorant et dispositifs électroluminescents.
